# EUROPEAN PATENT APPLICATION

(11) **EP 4 620 460 A1**
(43) Date of publication of application: **24.09.2025**
(21) Application number: 23891847.8
(22) Date of filing: 20.10.2023
(51) Int. Cl.: A61K 9/51, A61K 47/69, A61K 48/00

(54) **NANOPARTICLE COMPOSITION FOR DRUG DELIVERY**

(30) Priority: 16.11.2022 KR 20220153714
(71) Applicant: Samyang Holdings Corporation, Seoul 03129 (KR)
(72) Inventor: YOO, Seung Ju, Seoul 07943 (KR); LEE, So Jin, Seoul 02468 (KR); NAM, Joung Pyo, Seoul 02471 (KR); PARK, Jong Min, Seongnam-si Gyeonggi-do 13590 (KR); SEONG, Shi Hwa, Seoul 06008 (KR); JEONG, Seung Wei, Seongnam-si Gyeonggi-do 13568 (KR); HAN, Ji Hye, Seoul 03733 (KR)
(74) Representative: Gleiss Große Schrell und Partner mbB
(86) International application number: PCT/KR2023/016417
(87) International publication number: WO 2024/106780

(57) **Abstract**

The present invention relates to a nanoparticle composition for drug delivery and, more specifically, to a composition for drug delivery, wherein the composition comprises an amphiphilic block copolymer and a lipid with a specific structure that can easily form complexes with anionic drugs, whereby the composition can easily form drug-containing nanoparticles and thus is useful for drug delivery, especially for targeted delivery of drugs to the lungs.

## Description

### TECHNICAL FIELD

The present invention relates to a nanoparticle composition for drug delivery, and more specifically, a composition for drug delivery which comprises amphiphilic block copolymer and lipid with a specific structure capable of easily forming a complex with anionic drug, and can easily form drug-containing nanoparticle, and thus is useful for drug delivery, particularly for delivering drug specifically to lung.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, polycation polymers have cytotoxicity due to their multivalent cationic charges, making them problematic for practical use, and nucleic acid-cationic lipid complexes have low stability in the blood, making them difficult to use *in vivo* actually. In addition, ionic liposomes including cationic lipid, neutral lipid, and fusogenic lipid have the disadvantages of complication in method of synthesizing the cationic lipid used, cytotoxicity, and low efficiency of intracellular nucleic acid delivery.

In order to provide a mixed polymer nanoparticle composition capable of solubilizing a large amount of poorly water-soluble drug and having good stability in aqueous solution, Korean Laid-open Patent Publication No. 10-2003-0032897 discloses a mixed polymer nanoparticle composition which comprises an amphiphilic block copolymer of a hydrophilic block and a hydrophobic block, and a polylactic acid derivative containing carboxyl acid terminal group, and can form polymeric nanoparticles in body fluid or aqueous solution, and a pharmaceutical composition comprising polymeric nanoparticles consisting of the mixed polymer nanoparticle composition within which a poorly water-soluble drug is contained.

### CONTENTS OF THE INVENTION

### PROBLEMS TO BE SOLVED

The purpose of the present invention is to provide a composition useful for drug delivery, particularly useful for delivering drug specifically to lung.

### TECHNICAL MEANS

The first aspect of the present invention provides a composition for drug delivery comprising: drug as effective ingredient; and a lipid having a structure selected from the following, or an ionized form thereof: 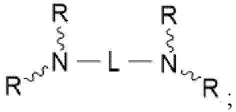 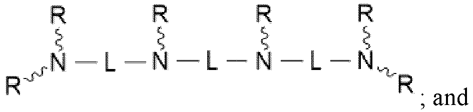 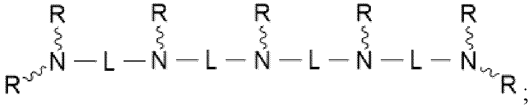
wherein, in each of the above structures,
at least two of R groups are Rx, and other R groups are Ry, wherein
each Rx is independently selected from 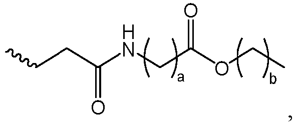 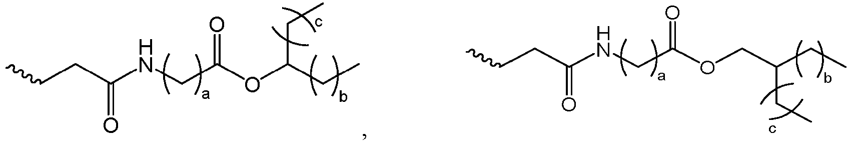 and 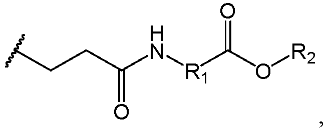 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 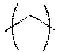 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

According to an embodiment of the present invention, each Ry may be independently H or C₁₋₂₀ alkyl group, where the alkyl group may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

According to an embodiment of the present invention, each L is independently C₁₋₂₀ alkylene group, each of which may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where the heterocyclic group may have one or more (e.g., 1 to 3) heteroatoms selected from N, O and S.

More concretely, each Rx is independently selected from 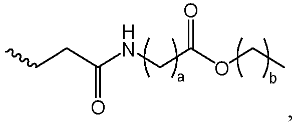 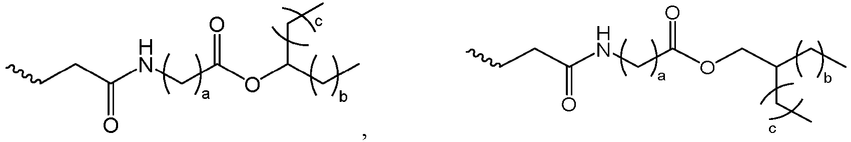 and 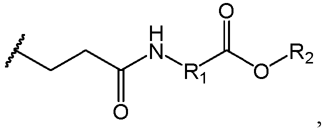 where each of a, b and c may be independently an integer of from 2 to 20 or from 2 to 15, R₁ may be substituted or unsubstituted, saturated or unsaturated divalent C₁₋₁₂ hydrocarbon group, R₂ may be substituted or unsubstituted, unsaturated monovalent C₂₋₂₄ hydrocarbon group, and 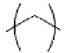 represents substituted or unsubstituted methylene group.

More concretely, each Ry may be independently H or C₁₋₁₀ alkyl group, where the alkyl group may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

More concretely, each L may be independently C₁₋₁₀ alkylene group, each of which may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S.

Even more concretely, each Rx is independently selected from 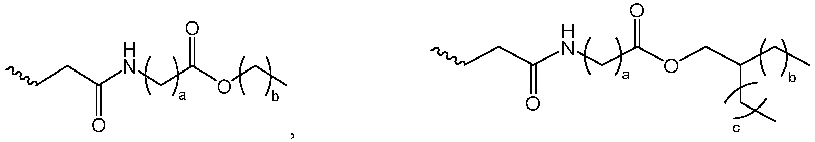 and 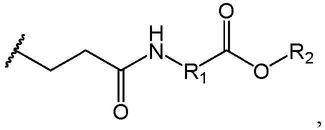 where each of a, b and c may be independently an integer of from 3 to 12, R₁ may be substituted or unsubstituted C₁₋₁₂ alkylene group, substituted or unsubstituted C₂₋₁₂ alkenylene group or substituted or unsubstituted C₂₋₁₂ alkynylene group, R₂ may be substituted or unsubstituted C₂₋₂₄ alkenyl group or substituted or unsubstituted C₂₋₂₄ alkynyl group, and 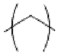 represents substituted or unsubstituted methylene group.

Even more concretely, each Ry may be independently H or C₁₋₆ alkyl group, where the alkyl group may be independently unsubstituted, or may be substituted with one or more selected from -OH and -NH₂; and two Ry groups that are not H may be connected together with nitrogen atom to which they are attached, to form a ring structure optionally having one or more heteroatoms selected from N and O.

Even more concretely, each L may be independently unsubstituted C₁₋₆ alkylene group.

Still more concretely, the lipid may be one having a structure selected from the following formulas A to V:

| Formula | Structure |
|---|---|
| A | 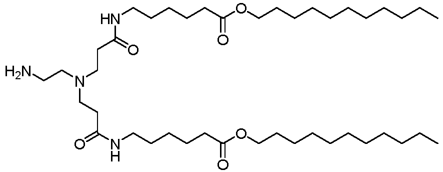 |
| B | 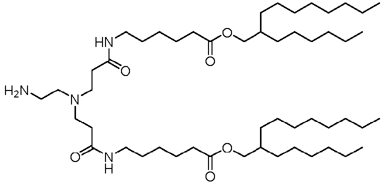 |
| C | 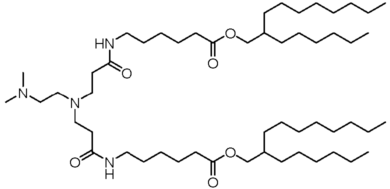 |
| D | 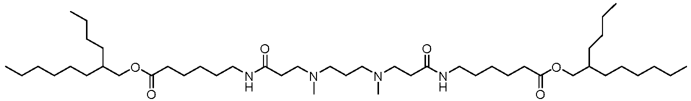 |
| E | 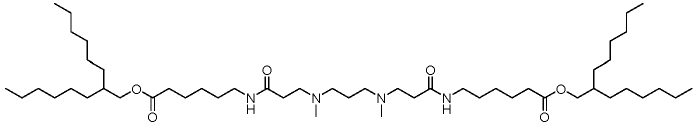 |
| F | 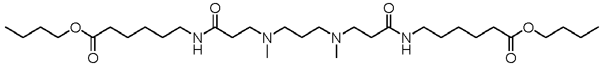 |
| G | 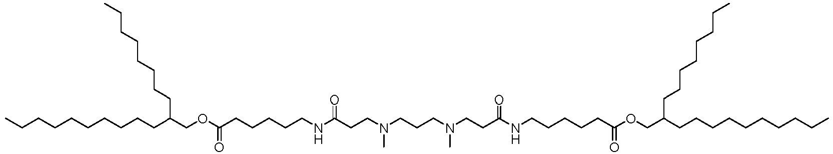 |
| H | 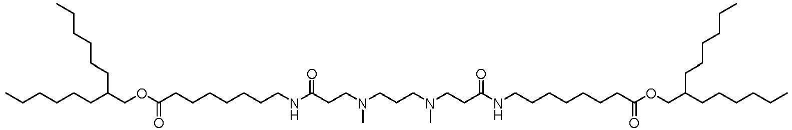 |
| I | 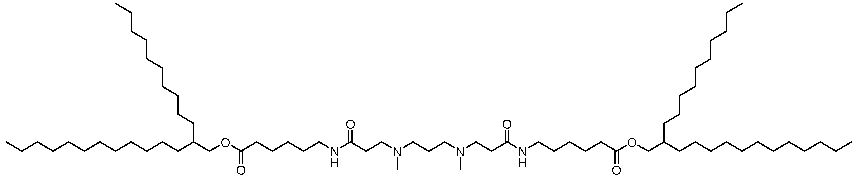 |
| J | 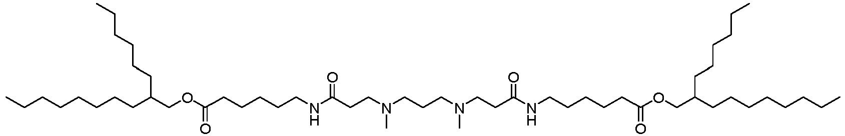 |
| K | 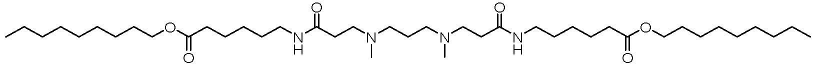 |
| L | 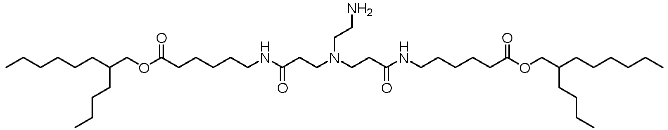 |
| M | 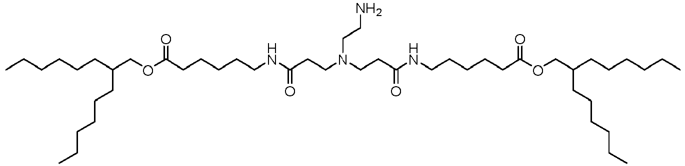 |
| N | 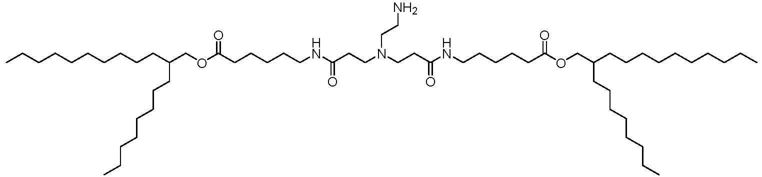 |
| O | 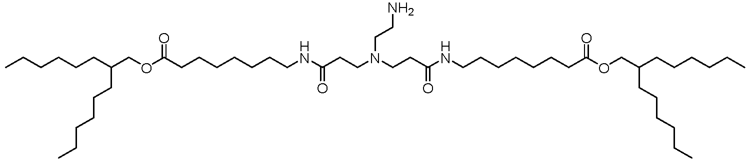 |
| P | 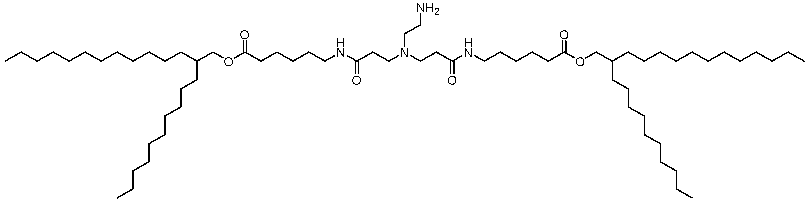 |
| Q | 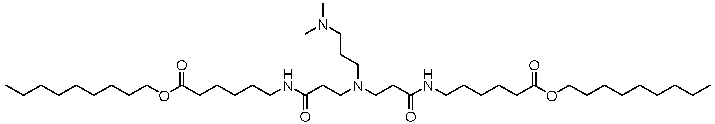 |
| R | 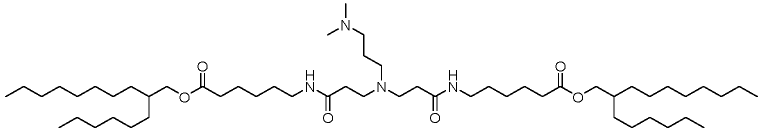 |
| S | 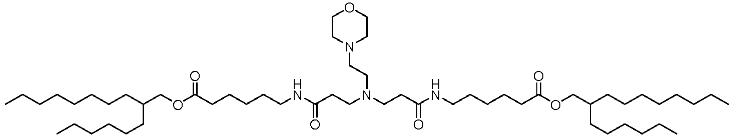 |
| T | 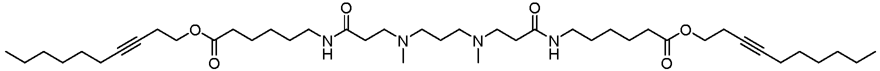 |
| U | 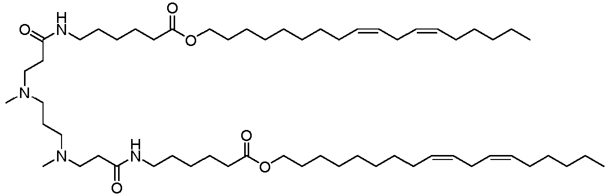 |
| V | 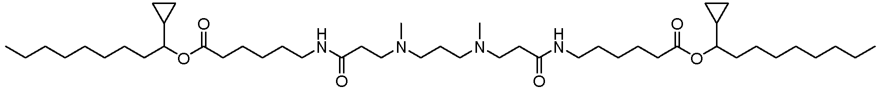 |

In an embodiment, the drug may form a complex with the lipid.

In an embodiment, the composition of the first aspect of the present invention may further comprise amphiphilic block copolymer.

In an embodiment, the drug forms a complex with the lipid, and the complex may be encapsulated within nanoparticle structure formed by the amphiphilic block copolymer.

In an embodiment, the composition of the first aspect of the present invention may be in a form of kit which comprises a first chamber comprising the lipid; and a second chamber comprising the drug.

In an embodiment, the first chamber may further comprise amphiphilic block copolymer.

The second aspect of the present invention provides a composition for drug delivery comprising nanoparticle, wherein the nanoparticle does not comprise drug, and wherein the nanoparticle comprises a lipid having a structure selected from the following, or an ionized form thereof: 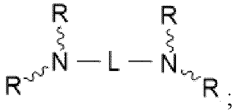 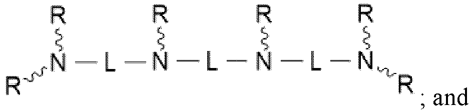 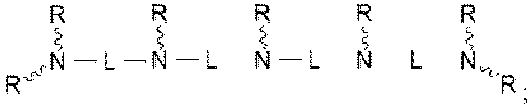 wherein R and L are the same as defined above.

In an embodiment, the nanoparticle may further comprise amphiphilic block copolymer.

In an embodiment, the composition for drug delivery of the present invention may be for delivering drug to lung.

In an embodiment, the composition for drug delivery of the present invention may further comprise salt of polylactic acid.

### EFFECT OF THE INVENTION

The composition according to the present invention is very useful for delivering drug in a form encapsulated within nanoparticle formed by amphiphilic block copolymer, specifically to lung.

### BRIEF EXPLANATION OF THE DRAWINGS

Figure 1 is a reaction scheme for the lipid synthesis procedure conducted in Preparation Example 1.
Figure 2 is a reaction scheme for the lipid synthesis procedure conducted in Preparation Example 2.
Figure 3 is a reaction scheme for the lipid synthesis procedure conducted in Preparation Example 3.
Figure 4 is a reaction scheme for the lipid synthesis procedure conducted in Preparation Example 4.
Figure 5 is a reaction scheme for the lipid synthesis procedure conducted in Preparation Example 5.
Figure 6 is a reaction scheme for the lipid synthesis procedure conducted in Preparation Example 6.
Figure 7 is a reaction scheme for the lipid synthesis procedure conducted in Preparation Example 7.
Figure 8 is a reaction scheme for the lipid synthesis procedure conducted in Preparation Example 8.
Figure 9 is a reaction scheme for the lipid synthesis procedure conducted in Preparation Example 9.
Figure 10 is a reaction scheme for the lipid synthesis procedure conducted in Preparation Example 10.
Figure 11 is a reaction scheme for the lipid synthesis procedure conducted in Preparation Example 11.
Figure 12 is a reaction scheme for the lipid synthesis procedure conducted in Preparation Example 12.
Figure 13 is a reaction scheme for the lipid synthesis procedure conducted in Preparation Example 13.
Figure 14 is a reaction scheme for the lipid synthesis procedure conducted in Preparation Example 14.
Figure 15 is a reaction scheme for the lipid synthesis procedure conducted in Preparation Example 15.
Figure 16 is a reaction scheme for the lipid synthesis procedure conducted in Preparation Example 16.
Figure 17 is a reaction scheme for the lipid synthesis procedure conducted in Preparation Example 17.
Figure 18 is a reaction scheme for the lipid synthesis procedure conducted in Preparation Example 18.
Figure 19 is a reaction scheme for the lipid synthesis procedure conducted in Preparation Example 19.
Figure 20 is a reaction scheme for the lipid synthesis procedure conducted in Preparation Example 20.
Figure 21 is a reaction scheme for the lipid synthesis procedure conducted in Preparation Example 21.
Figure 22 is a reaction scheme for the lipid synthesis procedure conducted in Preparation Example 22.
Figure 23 shows the results of a drug delivery experiment performed in the test example of the present invention and photographs of the results measured using a luminescence measurement imaging system.

### CONCRETE MODE FOR CARRYING OUT THE INVENTION

The present invention will be explained in detail below.

### [Lipid and preparation method thereof]

A lipid used in the present invention has a structure selected from the following, or is an ionized form thereof: 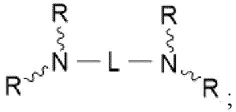 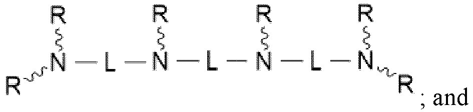 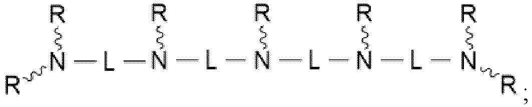
wherein, in each of the above structures,
at least two (more concretely, 2 to 7) of R groups are Rx, and other R groups are Ry, wherein
each Rx is independently selected from 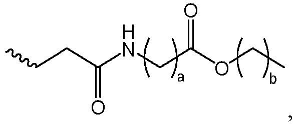 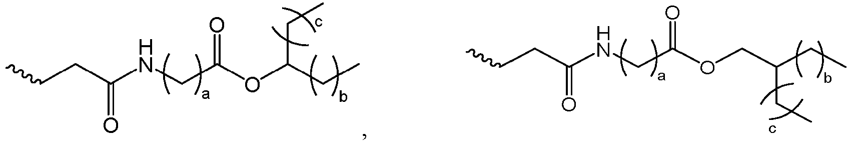 and 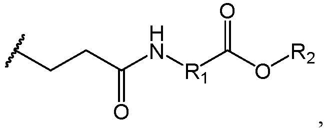 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 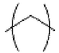 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

As used herein, the expression "substituted or unsubstituted" for any group means that, unless specified otherwise, the group is not substituted, or is substituted with one or more substituents selected from -OH, halogen atom, C₁₋₆ alkyl group, C₁₋₆ alkoxy group, C₁₋₆ halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

According to an embodiment of the present invention, each Rx is independently selected from 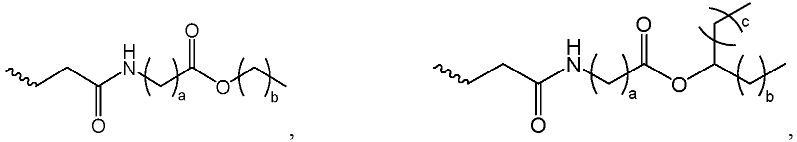 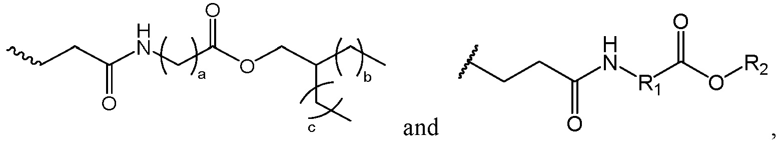 where each of a, b and c may be independently an integer of from 2 to 20 or from 2 to 15, R₁ may be substituted or unsubstituted, saturated or unsaturated divalent C₁₋₁₂ hydrocarbon group, R₂ may be substituted or unsubstituted, unsaturated monovalent C₂₋₂₄ hydrocarbon group, and 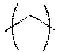 represents substituted or unsubstituted methylene group.

According to an embodiment of the present invention, each of a, b and c may be independently an integer of from 2 to 15, and more concretely, may be independently an integer of from 3 to 12. Even more concretely, a may be independently an integer of from 5 to 7, and each of b and c may be independently an integer of from 3 to 11, but it is not limited thereto.

In an embodiment, each Ry may be independently H or C₁₋₂₀ alkyl group, where the alkyl group may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O. Also, in the above, the alkyl or alkoxy group may be more concretely C₁₋₁₀ alkyl or alkoxy group, and still more concretely C₁₋₆ alkyl or alkoxy group, but it is not limited thereto.

In an embodiment, each L may be independently C₁₋₂₀ alkylene group (more concretely C₁₋₁₀ alkylene group, and still more concretely C₁₋₆ alkylene group), each of which may be independently unsubstituted, or may be substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group (e.g., C₃₋₂₀ cycloalkyl group or C₆₋₂₀ aryl group) and optionally substituted C₃₋₂₀ heterocyclic group (e.g., C₃₋₂₀ heterocycloalkyl group or C₃₋₂₀ heteroaryl group), where the heterocyclic group may have one or more (e.g., 1 to 3) heteroatoms selected from N, O and S. Also, in the above, the alkyl or alkoxy group may be more concretely C₁₋₁₀ alkyl or alkoxy group, and still more concretely C₁₋₆ alkyl or alkoxy group, but it is not limited thereto.

More concretely, each Ry may be independently H or C₁₋₁₀ alkyl group, where the alkyl group may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S; and two Ry groups that are not H may be connected together with nitrogen atom to which they are attached, to form a ring structure optionally having one or more heteroatoms selected from N and O.

More concretely, each L may be independently C₁₋₁₀ alkylene group, each of which may be independently unsubstituted, or may be substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where the heterocyclic group may have one or more (e.g., 1 to 3) heteroatoms selected from N, O and S.

Even more concretely, each Rx is independently selected from 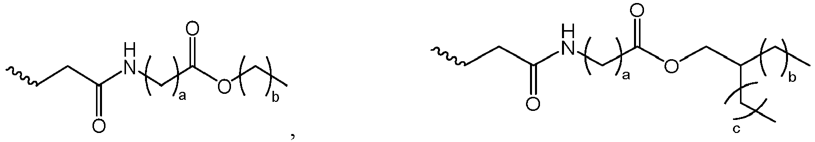 and 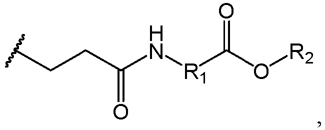 where each of a, b and c may be independently an integer of from 3 to 12, R₁ may be substituted or unsubstituted C₁₋₁₂ alkylene group, substituted or unsubstituted C₂₋₁₂ alkenylene group or substituted or unsubstituted C₂₋₁₂ alkynylene group, R₂ may be substituted or unsubstituted C₂₋₂₄ alkenyl group or substituted or unsubstituted C₂₋₂₄ alkynyl group, and 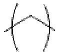 represents substituted or unsubstituted methylene group.

Even more concretely, each Ry may be independently H or C₁₋₆ alkyl group, where the alkyl group may be independently unsubstituted, or may be substituted with one or more selected from -OH and -NH₂; and two Ry groups that are not H may be connected together with nitrogen atom to which they are attached, to form a ring structure optionally having one or more heteroatoms selected from N and O.

Even more concretely, each L may be independently unsubstituted C₁₋₆ alkylene group.

Concretely, the lipid may have a structure selected from the following:

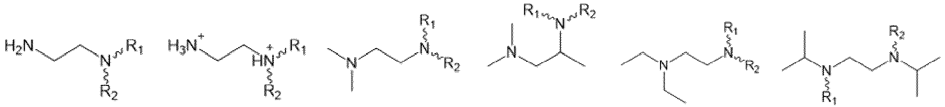

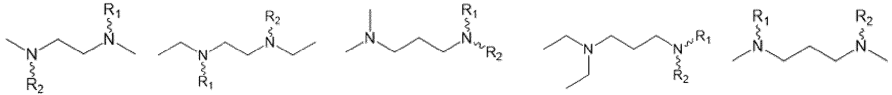

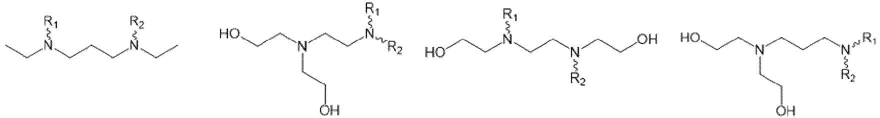

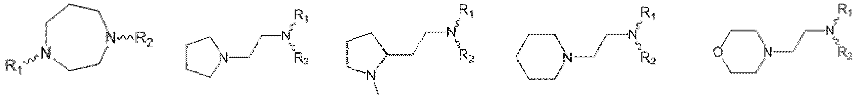

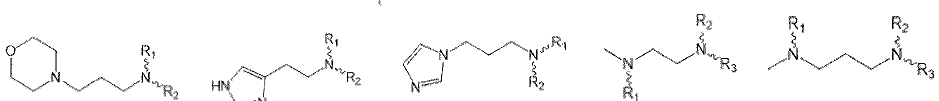

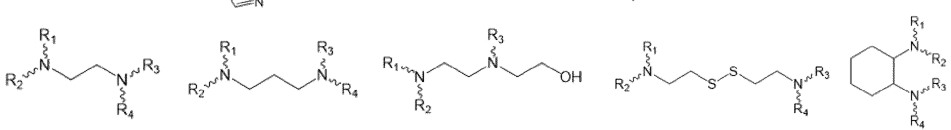

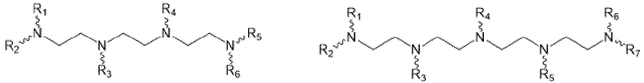

In each of the above structures, each of R₁ to R₇ is independently selected from 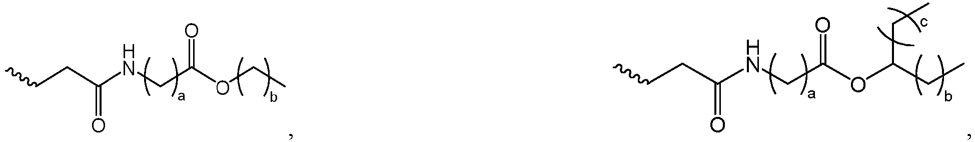 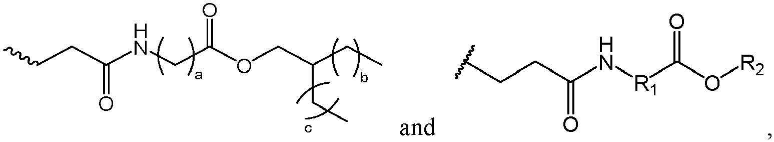 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 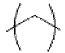 represents substituted or unsubstituted methylene group.

Still more concretely, the lipid may be one having a structure selected from the following formulas A to V:

| Formula | Structure |
|---|---|
| A | 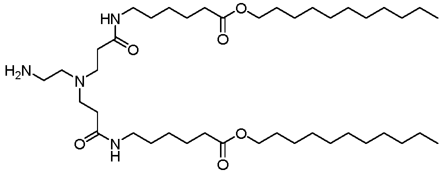 |
| B | 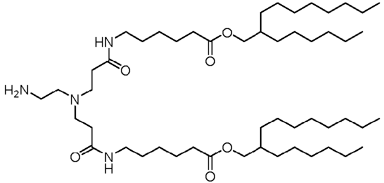 |
| C | 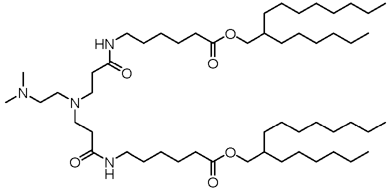 |
| D | 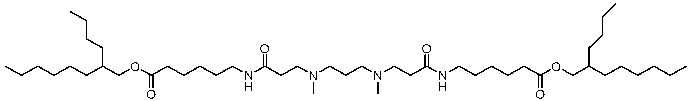 |
| E | 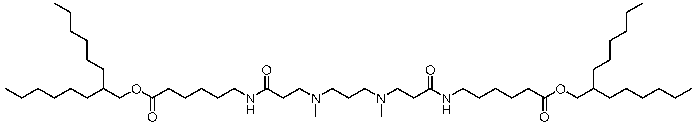 |
| F | 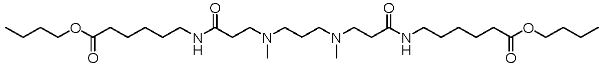 |
| G | 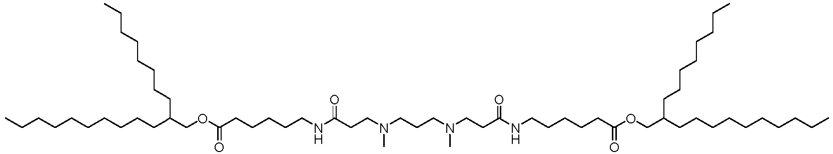 |
| H | 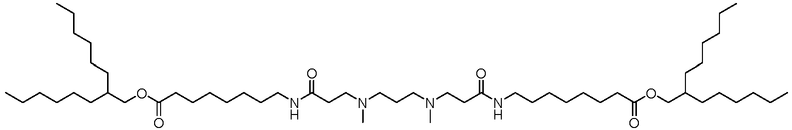 |
| I | 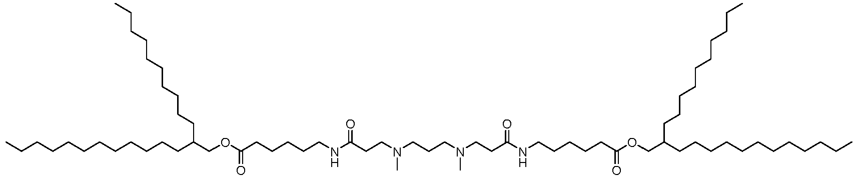 |
| J | 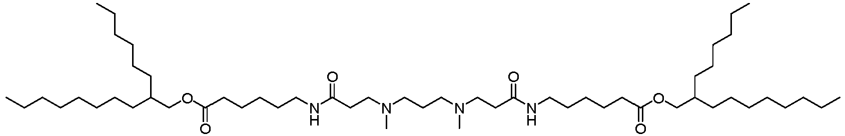 |
| K | 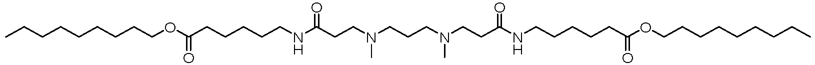 |
| L | 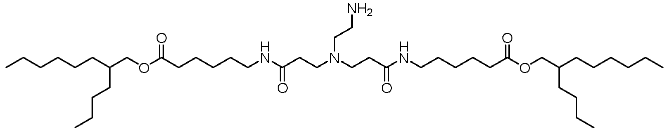 |
| M | 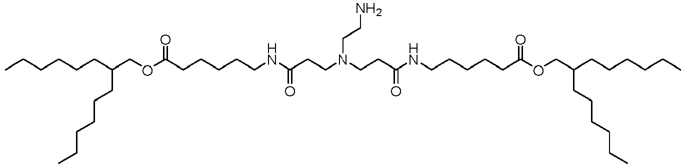 |
| N | 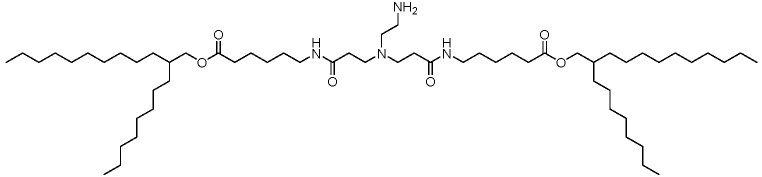 |
| O | 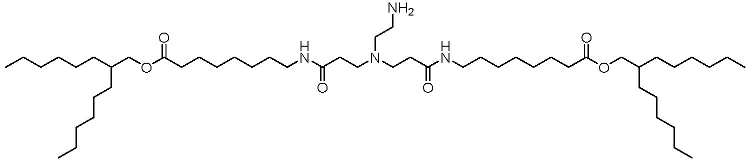 |
| P | 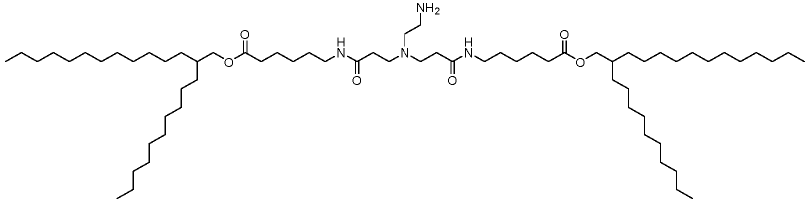 |
| Q | 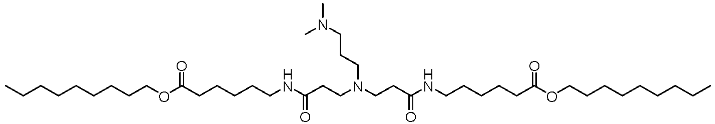 |
| R | 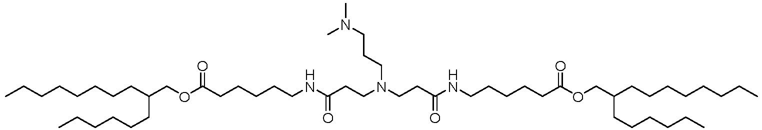 |
| S | 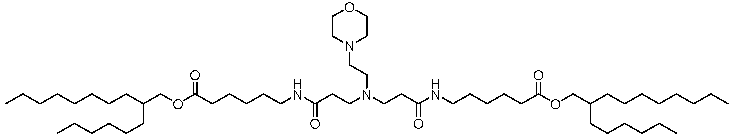 |
| T | 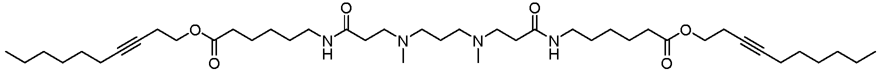 |
| U | 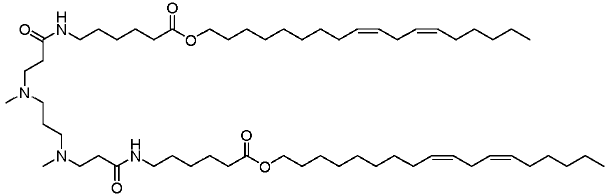 |
| V | 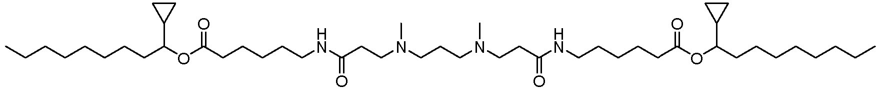 |

As an embodiment of the lipid used in the present invention, a lipid having a structure represented by the following formula (1-1) may be prepared by a method comprising steps of: (1) reacting a compound of formula (a) with a compound of formula (b) to obtain a compound of formula (c); (2) reacting a compound of formula (c) with a compound of formula (d) to obtain a compound of formula (e); and (3) reacting a compound of formula (e) with a compound of formula (f) and deprotecting the reaction product:

[Formula a] H₂N-(CH₂)ₐ-C(=O)OH

[Formula b] OH-R'

[Formula c] H₂N-(CH₂)ₐ-C(=O)O-R'

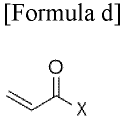

[Formula e] H₂C=CH-C(=O)-HN-(CH₂)ₐC(=O)O-R'

[Formula f] H₂N-(CH₂)₁₋₂₀-NH-C(=O)O-C(CH₃)₃

[Formula 1-1] H₂N-(CH₂)₁₋₂₀-N[-CH₂-CH₂-C(=O)-HN-(CH₂)ₐ-C(=O)O-R']₂

wherein,
each R' is independently 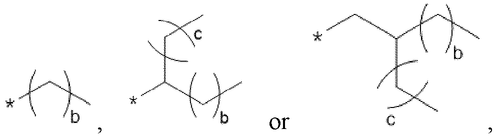 where * indicates the point of attachment to the adjacent oxygen atom, and 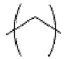 represents substituted or unsubstituted methylene group,
each of a, b and c is independently an integer of from 2 to 20, and
X is selected from the group consisting of F, CI, Br and I.

In an embodiment of the method for preparing a lipid having a structure represented by the above formula (1-1), the reaction of step (1) may be conducted in a solvent (e.g., cyclohexane) in the presence of a catalyst (e.g., p-toluenesulfonic acid monohydrate (p-TsOH)) under reflux, the reaction of step (2) may be conducted in a solvent (e.g., methylene chloride (MC)) in the presence of a catalyst (e.g., triethylamine (TEA)) at a low temperature (e.g., from -10°C to 10°C) or room temperature (e.g., from 20°C to 30°C) condition, the reaction of step (3) may be conducted in a solvent (e.g., n-butanol (n-BuOH)) under reflux, and the deprotection of step (3) may be conducted in a solvent (e.g., methylene chloride (MC)) in the presence of an acid (e.g., trifluoroacetic acid (TFA)) at room temperature (e.g., from 20°C to 30°C) condition, but it is not limited thereto.

As an embodiment of the lipid used in the present invention, a lipid having a structure represented by the following formula (1-2) may be prepared by a method comprising a step of reacting a compound of formula (e) obtained in the method for preparing a lipid having a structure represented by the above formula (1-1) with a compound of formula (g):

[Formula e] H₂C=CH-C(=O)-HN-(CH₂)ₐC(=O)O-R'

[Formula g] H₂N-(CH₂)₁₋₂₀-N(C₁₋₂₀alkyl)₂

[Formula 1-2] (C₁₋₂₀alkyl)₂N-(CH₂)₁₋₂₀-N[-CH₂-CH₂-C(=O)-HN-(CH₂)ₐ-C(=O)O-R']₂

wherein,
each R' is independently 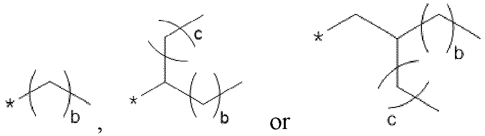 , where * indicates the point of attachment to the adjacent oxygen atom, and 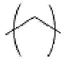 represents substituted or unsubstituted methylene group, and
each of a, b and c is independently an integer of from 2 to 20.

In an embodiment of the method for preparing a lipid having a structure represented by the above formula (1-2), the reaction may be conducted in a solvent (e.g., n-butanol (n-BuOH)) under reflux, but it is not limited thereto.

As an embodiment of the lipid used in the present invention, a lipid having a structure represented by the following formula (1-3) may be prepared by a method comprising a step of reacting a compound of formula (e) obtained in the method for preparing a lipid having a structure represented by the above formula (1-1) with a compound of formula (h):

[Formula e] H₂C=CH-C(=O)-HN-(CH₂)ₐC(=O)O-R'

[Formula h] (C₁₋₁₀alkyl)-NH-(CH₂)₁₋₂₀-NH-(C₁₋₁₀ alkyl)

[Formula 1-3] A-N(C₁₋₁₀alkyl)-(CH₂)₁₋₂₀-N(C₁₋₁₀alkyl)-A

wherein,
each R' is independently 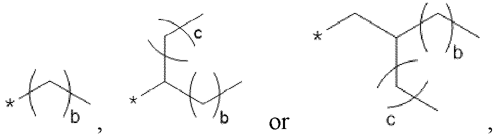 where * indicates the point of attachment to the adjacent oxygen atom, and 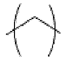 represents substituted or unsubstituted methylene group,
A is -CH₂-CH₂-C(=O)-HN-(CH₂)ₐ-C(=O)O-R',
each of a, b and c is independently an integer of from 2 to 20, and
X is selected from the group consisting of F, CI, Br and I.

In an embodiment of the method for preparing a lipid having a structure represented by the above formula (1-3), the reaction may be conducted in a solvent (e.g., n-butanol (n-BuOH)) under reflux, but it is not limited thereto.

As an embodiment of the lipid used in the present invention, a lipid having a structure represented by the following formula (1-4) may be prepared by a method comprising steps of: (1) reacting a compound of formula (a) with a compound of formula (b') to obtain a compound of formula (c'); (2) reacting a compound of formula (c') with a compound of formula (d) to obtain a compound of formula (e'); and (3) reacting a compound of formula (e') with a compound of formula (h):

[Formula a] H₂N-(CH₂)ₐ-C(=O)OH

[Formula b'] OH-R₂

[Formula c'] H₂N-R₁-C(=O)O-R₂

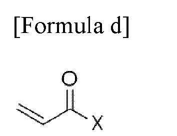

[Formula e'] H₂C=CH-C(=O)-HN-R₁-C(=O)O-R₂

[Formula h] (C₁₋₁₀alkyl)-NH-(CH₂)₁₋₂₀-NH-(C₁₋₁₀alkyl)

[Formula 1-4] A'-N(C₁₋₁₀alkyl)-(CH₂)₁₋₂₀-N(C₁₋₁₀ alkyl)-A'

wherein,
A' is -CH₂-CH₂-C(=O)-HN-R₁-C(=O)O-R₂,
R₁ is independently substituted or unsubstituted, saturated or unsaturated divalent hydrocarbon group,
R₂ is independently substituted or unsubstituted, unsaturated monovalent hydrocarbon group,
a is an integer of from 2 to 20, and
X is selected from the group consisting of F, CI, Br and I.

In an embodiment of the method for preparing a lipid having a structure represented by the above formula (1-4), the reaction of step (1) may be conducted in a solvent (e.g., cyclohexane) in the presence of a catalyst (e.g., p-toluenesulfonic acid monohydrate (p-TsOH)) under reflux, the reaction of step (2) may be conducted in a solvent (e.g., methylene chloride (MC)) in the presence of a catalyst (e.g., triethylamine (TEA)) at a low temperature (e.g., from -10°C to 10°C) or room temperature (e.g., from 20°C to 30°C) condition, and the reaction of step (3) may be conducted in a solvent (e.g., n-butanol (n-BuOH)) under reflux, but it is not limited thereto.

The lipid having the above specific structure used in the present invention can easily form a complex with anionic drug and thus is useful for drug delivery.

### [Composition for drug delivery]

The first aspect of the present invention relates to a composition for drug delivery comprising: drug as effective ingredient; and a lipid which has the above-explained specific structure, or is an ionized form thereof.

In an embodiment, the drug may form a complex with the lipid.

In an embodiment, the composition of the first aspect of the present invention may further comprise amphiphilic block copolymer.

In an embodiment, the drug forms a complex with the lipid, and the complex may be encapsulated within nanoparticle structure formed by the amphiphilic block copolymer.

In an embodiment, the composition of the first aspect of the present invention may be in a form of kit which comprises a first chamber comprising the lipid; and a second chamber comprising the drug.

In an embodiment, the first chamber may further comprise amphiphilic block copolymer.

The second aspect of the present invention relates to a composition for drug delivery comprising nanoparticle, wherein the nanoparticle comprises a lipid which has the above-explained specific structure, or is an ionized form thereof, and wherein the nanoparticle does not comprise drug.

In an embodiment, the nanoparticle may further comprise amphiphilic block copolymer.

The composition for drug delivery according to the second aspect of the present invention is not influenced by the storage or transportation environment, and when using it, an end user can quickly prepare drug-containing nanoparticles, even if the kind of drug (e.g., mRNA) is changed, only by simple mixing of the composition and the drug without complicated process, and accordingly, for example, in case of personalized vaccine or pandemic situation, it is not necessary to optimize the production process with mRNA in hospital, and thus once the complete mRNA is produced, it can be simply mixed with the composition of the present invention and promptly administered into human body.

In an embodiment, the composition for drug delivery of the present invention may be for delivering drug to lung.

In an embodiment, the composition for drug delivery of the present invention may further comprise salt of polylactic acid.

In an embodiment, the drug forms a complex with the lipid, and the complex may be encapsulated within nanoparticle structure formed by the amphiphilic block copolymer and the salt of polylactic acid.

In the nanoparticle, in an aqueous environment, the hydrophilic part of the amphiphilic block copolymer forms an outer wall of the nanoparticle and the hydrophobic part of the amphiphilic block copolymer (together with the salt of polylactic acid if it is further contained) forms an inner wall of the nanoparticle, and inside the nanoparticle formed as such, the complex of the drug and the lipid can be encapsulated. Such a nanoparticle structure enhances the stability of the effective ingredient in blood or body fluid.

In an embodiment, the drug may be selected from nucleic acid, polypeptide, virus or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

In an embodiment, the particle size of the nanoparticle can be defined by Z-average value, and for example, it may be 800 nm or less, 600 nm or less, 500 nm or less, 400 nm or less, 300 nm or less, 200 nm or less, or 150 nm or less, and also may be 10 nm or more, 50 nm or more, or 100 nm or more. In a concrete embodiment, the particle size of the nanoparticle defined by Z-average value may be, for example, 10 to 800 nm, 20 to 600 nm, 30 to 500 nm, 50 to 400 nm, or 80 to 300 nm.

The "Z-average" may mean the average of hydrodynamic diameter of particle distribution measured by using Dynamic light scattering (DSL). The nanoparticles have monodisperse particle distribution, and the polydispersity index thereof may be, for example, 0.01 to 0.50, 0.05 to 0.455, or 0.1 to 0.40.

Also, in an embodiment, the surface charge of the nanoparticle may be, for example, 0 mV or more, 1 mV or more, 5 mV or more, or 10 mV or more, and also may be 80 mV or less, 70 mV or less, or 60 mV or less. In a concrete embodiment, the surface charge of the nanoparticle may be, for example, 0 to 80 mV, 1 to 70 mV, or 5 to 60 mV. The surface charge may be measured in an environment close to biological environment, and for example, it may be measured in 8 to 12 mM HEPES buffer (pH 7.0 to 7.5).

In case of maintaining the particle size and surface charge of the nanoparticle to the above level, it is preferable in terms of stability of nanoparticle structure, amounts of the components and absorbability in the body, and easiness of sterilization as a pharmaceutical composition. For example, in case where the effective ingredient is nucleic acid, the one or more ends of the nucleic acid may be modified with one or more selected from the group consisting of cholesterol, tocopherol and fatty acids having 10 to 24 carbon atoms. The cholesterol, tocopherol and fatty acids having 10 to 24 carbon atoms include each analogue, derivative and metabolite of the cholesterol, tocopherol and fatty acids.

In an embodiment, if the effective ingredient is RNA, the amount of the effective ingredient may be, based on the total weight of the nanoparticle, for example, 0.05 to 30 % by weight, 0.1 to 25 % by weight, 0.25 to 20 % by weight, 0.5 to 15 % by weight, 1 to 10 % by weight, or 1 to 5 % by weight. If the amount of the effective ingredient is less than the above range, the amount of delivery carrier becomes too much as compared with the drug, and thus there may be a side effect due to the delivery carrier. If the amount of the effective ingredient is greater than the above range, the size of the nanoparticle becomes too large, and thus the nanoparticle stability may be lowered and the rate of loss during filter sterilization may increase.

In an embodiment, the amount of the lipid may be, based on 1 part by weight of the effective ingredient, for example, 500 parts by weight or less, 400 parts by weight or less, 300 parts by weight or less, 200 parts by weight or less, 100 parts by weight or less, 80 parts by weight or less, or 60 parts by weight or less, and it also may be 1 part by weight or more, 2 parts by weight or more, 3 parts by weight or more, 4 parts by weight or more, or 5 parts by weight or more. In an embodiment, the amount of the lipid may be, based on 1 part by weight of the effective ingredient, 1 to 500 parts by weight, 2 to 400 parts by weight, 3 to 300 parts by weight, 4 to 200 parts by weight, or 5 to 80 parts by weight. If the amount of the lipid is less than the above range, it may not form a stable complex with the effective ingredient. If the amount of the lipid is greater than the above range, the size of the nanoparticle becomes too large, and thus the nanoparticle stability may be lowered and the rate of loss during filter sterilization may increase.

In case where the effective ingredient is nucleic acid, the lipid and the nucleic acid are combined together through electrostatic interaction to form a complex. In an embodiment, the ratio of quantities of electric charge of the lipid (N) and the nucleic acid (P) (N/P: the ratio of the positive electric charge of the lipid to the negative electric charge of the nucleic acid) may be 0.5 or more, 1 or more, 2 or more, or 5 or more, and it also may be 200 or less, 150 or less, 100 or less, or 60 or less, and for example, it may be 0.5 to 200, 1 to 150, 2 to 100, or 5 to 60. If the ratio (N/P) is less than the above range, it may be difficult to form a complex containing a sufficient amount of the nucleic acid. **In** contrast, if the ratio (N/P) is greater than the above range, toxicity may be induced. **In** addition, the N/P ratio may act importantly for expression of the effective ingredient specifically in spleen.

**In** a concrete embodiment, the amphiphilic block copolymer may be an A-B type block copolymer comprising a hydrophilic A block and a hydrophobic B block. **In** an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

In an embodiment, the number average molecular weight (g/mol) of the hydrophilic A block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and glycolide, copolymer of polylactide and polydioxan-2-one, copolymer of polylactide and and polycaprolactone, and a copolymer of polyglycolide and polycaprolactone.

In an embodiment, the number average molecular weight (g/mol) of the hydrophobic B block may be 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic A block-hydrophobic B block may be 2,000-6,000, 2,000-4,000, 2,000-3,000, 2,000-1,700, etc., but it is not limited thereto.

Also, in an embodiment, in order to increase the hydrophobicity of and thereby improve the stability of the nanoparticle, the hydrophobic B block may be modified by chemically combining the hydroxyl group at the end of the hydrophobic B block with tocopherol, cholesterol, or fatty acid having 10 to 24 carbons.

In an embodiment, the salt of polylactic acid is distributed in the core (inner wall) of the nanoparticle, and acts to stabilize the nanoparticle by strengthening the hydrophobicity of the core, and at the same time, to effectively avoid reticuloendothelial system (RES) in the body. That is, the carboxylic anion in the salt of polylactic acid binds to the cationic complex more efficiently than polylactic acid, and decreases the surface potential of the polymer nanoparticle. Thereby, positive charge of the surface potential of the polymer nanoparticle becomes less than that of a polymer nanoparticle which does not contain a salt of polylactic acid, and thus it may be less captured by reticuloendothelial system and efficiently delivered to target sites (e.g., cancer cells, inflammatory cells, etc.).

In an embodiment, the number average molecular weight (g/mol) of the salt of polylactic acid may be 500 to 50,000, and more concretely, 1,000 to 10,000. If the number average molecular weight of the salt of polylactic acid is less than 500, the hydrophobicity becomes too low and thus it may not exist in the core (inner wall) of the nanoparticle, and if the number average molecular weight of the salt of polylactic acid is greater than 50,000, the size of the polymer nanoparticle may become too large.

In an embodiment, the end of the salt of polylactic acid (e.g., sodium salt of polylactic acid) opposite to the end of carboxylic acid-metal (e.g., carboxylic acid-sodium) may be substituted with one selected from the group consisting of hydroxyl, acetoxy, benzoyloxy, decanoyloxy, palmitoyloxy, and alkoxy having 1 to 2 carbon atoms.

In an embodiment, the salt of polylactic acid may be one or more selected from the group consisting of the compounds of the following Formulas 2 to 7 (wherein "COO" means carboxylic group, i.e., "C(=O)O"):

[Formula 2] **RO-CHZ-[A]ₙ-[B]ₘ-COOM**

In Formula 2 above, A is -COO-CHZ-; B is -COO-CHY-, -COO-CH₂CH₂CH₂CH₂CH₂- or -COO-CH₂CH₂OCH₂-; R is hydrogen atom, or acetyl, benzoyl, decanoyl, palmitoyl, methyl or ethyl; each of Z and Y is hydrogen atom, or methyl or phenyl; M is Na, K or Li; n is an integer of from 1 to 30; and m is an integer of from 0 to 20.

[Formula 3] **RO-CHZ-[COO-CHX]ₚ-[COO-CHY']_{q}-COO-CHZ-COOM**

In Formula 3 above, X is methyl; Y' is hydrogen atom or phenyl; p is an integer of from 0 to 25, q is an integer of from 0 to 25, with the proviso that p + q is an integer of from 5 to 25; R is hydrogen atom, or acetyl, benzoyl, decanoyl, palmitoyl, methyl or ethyl; M is Na, K or Li; and Z is hydrogen atom, methyl or phenyl.

[Formula 4] **RO-PAD-COO-W-M'**

In Formula 4 above, W-M' is 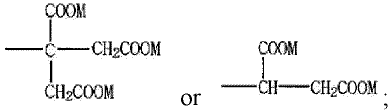 PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; R is hydrogen atom, or acetyl, benzoyl, decanoyl, palmitoyl, methyl or ethyl; and M is independently Na, K or Li.

[Formula 5] **S-O-PAD-COO-Q**

In Formula 5 above, S is 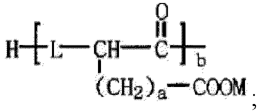 L is -NR₁- or -O-, wherein R₁ is hydrogen atom or C₁₋₁₀ alkyl; Q is -CH₃, -CH₂CH₃, -CH₂CH₂CH₃, -CH₂CH₂CH₂CH₃, or -CH₂C₆H₅; a is an integer of from 0 to 4; b is an integer of from 1 to 10; M is Na, K or Li; and PAD is one or more selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one.

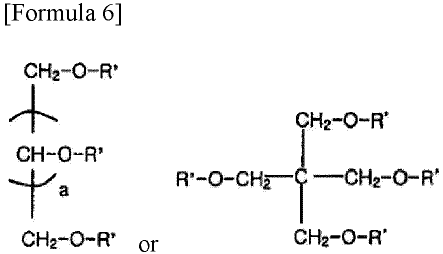

In Formula 6 above, R' is -PAD-O-C(O)-CH₂CH₂-C(O)-OM, wherein PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one, M is Na, K or Li; and a is an integer of from 1 to 4.

[Formula 7] **YO-[-C(O)-(CHX)ₐ₋O-]ₘ-C(O)-R-C(O)-[-O-(CHX')_{b}-C(O)-]ₙ-OZ**

In Formula 7 above, X and X' are independently hydrogen, C₁₋₁₀ alkyl or C₆₋₂₀ aryl; Y and Z are independently Na, K or Li; m and n are independently an integer of from 0 to 95, with the proviso that 5 < m + n < 100; a and b are independently an integer of from 1 to 6; and R is -(CH₂)ₖ-, C₂₋₁₀ divalent alkenyl, C₆₋₂₀ divalent aryl or a combination thereof, wherein k is an integer of from 0 to 10.

In an embodiment, the salt of polylactic acid may be a compound of Formula 2 or Formula 3.

In an embodiment, in order to increase the efficiency of intracellular delivery of drug (e.g., mRNA), the composition of the present invention may further comprise fusogenic lipid.

When mixed in a complex of drug (e.g., mRNA) and the above lipid, the fusogenic lipid forms a complex of drug (e.g., mRNA)/the above lipid/fusogenic lipid by hydrophobic interaction, and the complex comprising the fusogenic lipid is encapsulated within the nanoparticle structure of the amphiphilic block copolymer.

More concretely, the fusogenic lipid may be one or more phospholipid selected from the group consisting of phosphatidylethanolamin (PE), phosphatidylcholine (PC) and phosphatidic acid. The phosphatidylethanolamin (PE), phosphatidylcholine (PC) and phosphatidic acid may be in a form combined with one or two C₁₀₋₂₄ fatty acids. Cholesterol and tocopherol include analogues, derivatives and metabolites of each of the cholesterol and tocopherol.

Still more concretely, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine (DOPE), 1,2-dipalmitoleoyl-sn-glycero-3-phosphoethanolamine (DPPE), dilinoleoyl phosphatidylethanolamine, 1-palmitoyl-2-oleoyl phosphatidylethanolamine, 1,2-diphytanoyl-3-sn-phosphatidylethanolamine, 1,2-dipalmitoleoyl-sn-glycero-3-phosphocholine (DPPC), 1,2-dioleoyl-sn-glycero-3-phosphocholine (DOPC), dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid. 1-palmitoyl-2-oleoyl phosphatidic acid, 1,2-diphytanoyl-3-sn-phosphatidic acid, cholesterol and tocopherol.

Still more concretely, the fusogenic lipid may be one or more selected from the group consisting of dioleoyl phosphatidylethanolamine (DOPE), 1,2-dipalmitoleoyl-sn-glycero-3-phosphocholine (DPPC), 1,2-dioleoyl-sn-glycero-3-phosphocholine (DOPC), distearoylphosphatidylcholine (DSPC) and 1,2-dipalmitoleoyl-sn-glycero-3-phosphoethanolamine (DPPE).

In an embodiment, the amount of the amphiphilic block copolymer used may be 1 to 500 parts by weight, 1 to 400 parts by weight, 1 to 300 parts by weight, 2 to 500 parts by weight, 2 to 400 parts by weight, 2 to 300 parts by weight, 3 to 500 parts by weight, 3 to 400 parts by weight, 3 to 300 parts by weight, 4 to 500 parts by weight, 4 to 400 parts by weight, or 4 to 300 parts by weight, based on 1 part by weight of the lipid.

In an embodiment, the amount of the salt of polylactic acid used may be 0.1 to 100 parts by weight, 0.1 to 80 parts by weight, 0.1 to 50 parts by weight, 0.1 to 30 parts by weight, 0.1 to 10 parts by weight, 0.1 to 5 parts by weight, 0.5 to 100 parts by weight, 0.5 to 80 parts by weight, 0.5 to 50 parts by weight, 0.5 to 30 parts by weight, 1 to 100 parts by weight, 1 to 80 parts by weight, 1 to 50 parts by weight, 1 to 30 parts by weight, 2 to 100 parts by weight, 2 to 80 parts by weight, 2 to 50 parts by weight, or 2 to 30 parts by weight, based on 1 part by weight of the lipid.

In an embodiment, the composition of the present invention may further comprise aqueous solution, water miscible organic solvent, or a combination thereof. The "aqueous solution" may be used for the same meaning as water solution, and may mean, for example, water, sterilized water, buffer solution, injection solution, etc., and it may be a buffer solution further comprising organic acid. The aqueous solution may be, for example, citric acid buffer, PBS buffer, etc., but it is not limited thereto. The "water miscible organic solvent" may be C1 to C4 lower alcohol, acetone, acetonitrile, a water mixture thereof or a mixture thereof, but it is not limited thereto.

In an embodiment, the composition of the present invention may further comprise a stabilizing agent suitable for improving stability of the effective ingredient. The stabilizing agent may further include pH adjusting agent, inorganic salt, saccharide, surfactant, chelating agent, etc., but it is not limited thereto. The "saccharide" may mean monosaccharide, disaccharide, sugar alcohol which is reduced sugar thereof, and polymer of single or mixed polysaccharides, etc., and the polysaccharide may mean tri- or more saccharide. For example, the monosaccharide may be mannose, glucose, arabinose, fructose, galactose, etc.; the disaccharide may be sucrose, trehalose, maltose, lactose, cellobiose, gentiobiose, isomaltose, melibose, etc.; the sugar alcohol may be mannitol, sorbitol, xylitol, erythritol, maltitol, etc.; and the polysaccharide may be raffinose, dextran, starch, hydroxyethyl starch, cyclodextrin, cellulose, hetastarch, oligosaccharide, etc. but it is not limited thereto. The "pH adjusting agent" may be Tris, glycine, histidine, glutamate, succinate, phosphate, acetate, aspartate, or a combination thereof, and the "surfactant" may be sodium lauryl sulfate, dioctyl sodium sulfosuccinate, dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, glycodeoxycholic acid sodium salt, benzalkonium chloride, Triton X-100, Triton X-114, lauromacrogol 400, polyoxyl 40 stearate, polysorbate 20, 40, 60, 65 or 80, or a combination thereof, but it is not limited thereto. The "chelating agent" may be citric acid, polyphenolic acid, EDTA, DTPA, EDDHA, or a combination thereof, but it is not limited thereto. The "inorganic salt" means a salt of monovalent or divalent metal and may be NaCl, KCl, MgCl₂, CaCl₂, MgSO₄, CaSO₄, CaCO₃, MgCO₃, etc., but it is not limited thereto.

The present invention will be explained below in more detail with reference to the following Examples. However, the Examples are only to illustrate the invention, and the scope of the present invention is not limited thereby in any manner.

### EXAMPLES

### [Preparation of lipid]

### Preparation Example 1

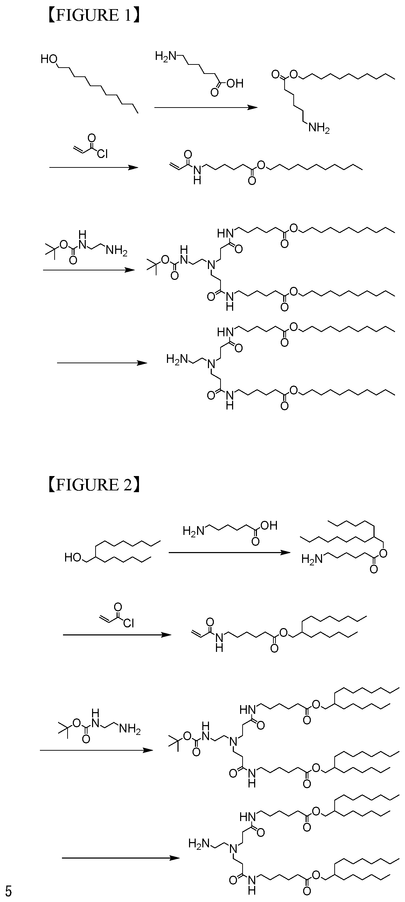

### 1-1. The compound of the following formula A was prepared according to the synthesis scheme shown in Figure 1.

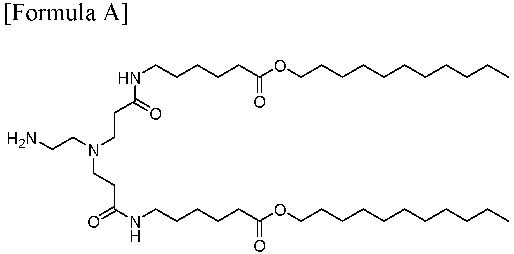

### 1-2. Synthesis of undecyl 6-acrylamidohexanoate

In a 250 mL 3-neck round bottom flask (RBF), 6-aminohexanoic acid (10 g, 76.23 mmol, 1.1 eq), undecan-1-ol (11.94 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the reaction product was concentrated in vacuo, extracted with methylene chloride (MC) and 3% sodium hydroxide aqueous solution, and the methylene chloride layer was dried over sodium sulfate and filtered. The filtrate was concentrated under vacuum to obtain undecyl 6-aminohexanoate with low purity. Without further purification, previously obtained undecyl 6-aminohexanoate, methylene chloride (100 mL), and triethylamine (TEA) (15.43 g, 152.46 mmol, 2.2 eq) were added to a 250 mL 3-neck RBF, cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with ethyl acetate:hexane (1:1) to obtain undecyl 6-acrylamidohexanoate (17.22 g, yield: 73%).
¹H-NMR (400 MHz, CDCl₃) *δ* 6.25 (dd, 1H), 6.01-6.08 (m, 1H), 5.61-5.68 (m, 2H), 4.01 (t, 2H), 3.31 (q, 2H), 2.28 (t, 2H), 1.43-1.68 (m, 6H), 1.30-1.45 (m, 18H), 0.89 (t, 3H)

### 1-3. Synthesis of undecyl 2,2-dimethyl-4,11-dioxo-8-(3-oxo-3-((6-oxo-6-(undecyloxy)hexyl)amino)propyl)-3-oxa-5,8,12-triazaoctadecan-18-oate

In a 100 mL 1-neck RBF, undecyl 6-acrylamidohexanoate (3 g, 8.83 mmol, 3 eq), tert-butyl (2-aminoethyl)carbamate (0.47 g, 2.94 mmol, 1 eq), and n-butanol (n-BuOH) (40 mL) were added, and then stirring and reflux were performed. After 4 days, the mixture was concentrated in vacuo at 70°C and purified using a silica column with methylene chloride:methanol:ammonium hydroxide (15:1:0.1) to obtain undecyl 2,2-dimethyl-4,11-dioxo-8-(3-oxo-3-((6-oxo-6-(undecyloxy)hexyl)amino)propyl)-3-oxa-5,8,12-triazaoctadecan-18-oate (1.34 g, yield: 54%).
¹H-NMR (400 MHz, CDCl₃) *δ* 4.06 (t, 4H), 3.49 (q, 4H), 3.26-3.16 (br, 2H), 2.71 (q, 4H), 2.48-2.41 (br, 2H), 2.32-2.85 (m, 8H), 1.67-1.26 (m, 48H), 1.44 (s, 9H), 0.87 (t, 6H)

### 1-4. Synthesis of the compound of formula A

In a 100 mL 1-neck RBF, undecyl 2,2-dimethyl-4,11-dioxo-8-(3-oxo-3-((6-oxo-6-(undecyloxy)hexyl)amino)propyl)-3-oxa-5,8,12- triazaoctadecan-18-oate (1 g, 1.19 mmol) and methylene chloride (20 mL) were added, and trifluoroacetic acid (TFA) (2 mL) was added dropwise. After stirring at room temperature for 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with methylene chloride:methanol:ammonium hydroxide (7:1:0.1) to obtain the compound of formula A (0.31 g, 35%).
¹H-NMR (400 MHz, CDCl₃) δ 6.86 (t, 2H), 4.06 (t, 4H), 3.25 (t, 4H), 2.89 (t, 2H), 2.68 (t, 4H), 2.58 (t, 2H), 2.36 (t, 4H), 2.31 (t, 2H), 1.65 - 1.26 (m, 48H), 0.89 (t, 6H)

### Preparation Example 2

### 2-1. The compound of the following formula B was prepared according to the synthesis scheme shown in Figure 2.

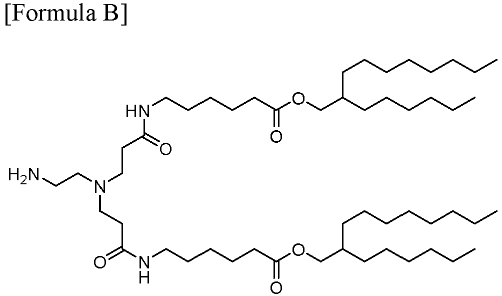

### 2-2. Synthesis of 2-hexyldecyl 6-acrylamidohexanoate

In a 250 mL 3-neck RBF, 6-aminohexanoic acid (10 g, 76.23 mmol, 1.1 eq), 2-hexyldecan-1-ol (16.80 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-hexyldecyl 6-aminohexanoate with low purity. Without further purification, previously obtained 2-hexyldecyl 6-aminohexanoate, methylene chloride (100 mL), and triethylamine (TEA) (15.43 g, 152.46 mmol, 2.2 eq) were added to a 250 mL 3-neck RBF, cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with ethyl acetate:hexane (1:1) to obtain 2-hexyldecyl 6-acrylamidohexanoate (14.25 g, yield: 50%).
¹H-NMR (400 MHz, CDCl₃) *δ* 6.27 (dd, 1H), 6.07-6.12 (m, 1H), 5.62-5.64 (m, 2H), 3.97 (d, 2H), 3.34 (q, 2H), 2.32 (t, 2H), 1.54-1.68 (m, 5H), 1.26-1.41 (m, 26H), 0.87 (t, 6H)

### 2-3. Synthesis of 2-hexyldecyl 8-(3-((6-((2-hexyldecyl)oxy)-6-oxohexyl)amino)-3-oxopropyl)-2,2-dimethyl-4,11-dioxo-3-oxa-5.8.12-triazaoctadecan-18-oate

In a 100 mL 1-neck RBF, 2-hexyldecyl 6-acrylamidohexanoate (4 g, 9.76 mmol, 3 eq), tert-Butyl (2-aminoethyl) carbamate (0.52 g, 3.25 mmol, 1 eq), and n-butanol (n-BuOH) (40 mL) were added, and then stirring and reflux were performed. After 4 days, the mixture was concentrated in vacuo at 70°C and purified using a silica column with methylene chloride:methanol:ammonium hydroxide (15:1:0.1) to obtain 2-hexyldecyl 8-(3-((6-((2-hexyldecyl)oxy)-6-oxohexyl)amino)-3-oxopropyl)-2,2-dimethyl-4,11-dioxo-3-oxa-5,8,12-triazaoctadecan-18-oate (2.23 g, yield: 70%).

### 2-4. Synthesis of the compound of formula B

In a 100 mL 1-neck RBF, 2-hexyldecyl 8-(3-((6-((2-hexyldecyl)oxy)-6-oxohexyl)amino)-3-oxopropyl)-2,2-dimethyl-4,11-dioxo-3-oxa-5,8,12-triazaoctadecan-18-oate (2 g, 2.04 mmol) and methylene chloride (40 mL) were added, and trifluoroacetic acid (TFA) (4 mL) was added dropwise. After stirring at room temperature for 4 hours, the mixture in the reactor was extracted with a saturated aqueous solution of sodium bicarbonate, and the methylene chloride layer was dried over sodium sulfate and filtered. The filtrate was concentrated in vacuo and purified using a silica column with methylene chloride:methanol:ammonium hydroxide (7:1:0.1) to obtain the compound of formula B (1.21 g, 67%).
¹H-NMR (400 MHz, CDCl₃) δ 6.96 (t, 2H), 3.96 (t, 4H), 3.21 - 3.13 (m, 6H), 2.71 (t, 2H), 2.64 (t, 4H), 2.37 (t, 4H), 2.31 (t, 4H), 1.65 - 1.33 (m, 62H), 0.89 (t, 6H)

### Preparation Example 3

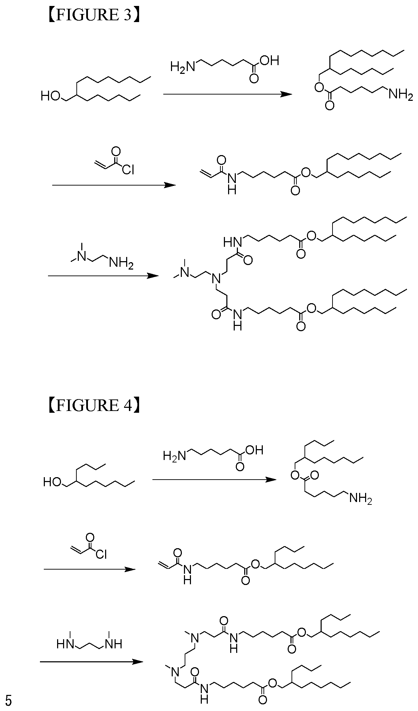

### 3-1. The compound of the following formula C was prepared according to the synthesis scheme shown in Figure 3.

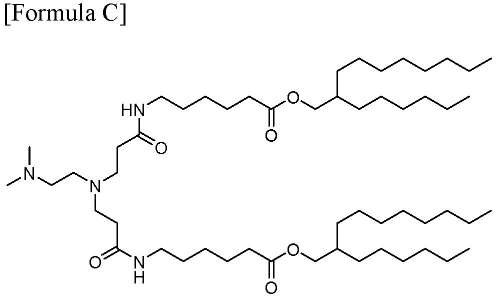

### 3-2. Synthesis of the compound of formula C

In a 100 mL 1-neck RBF, 2-hexyldecyl 6-acrylamidohexanoate (3 g, 7.32 mmol, 3 eq) synthesized in Example 2-2, N,N-dimethylethylenediamine (0.22 g, 2.44 mmol, 1 eq), and n-butanol (n-BuOH) (30 mL) were added, and then stirring and reflux were performed. After 3 days, the mixture was concentrated in vacuo at 70°C, and purified using a silica column with methylene chloride:methanol:ammonium hydroxide (10:1:0.1) to obtain the compound of formula C (0.95 g, yield: 43%).
¹H-NMR (400 MHz, CDCl₃) δ 4.05 (t, 4H), 3.15 (q, 4H), 2.77 (br, 4H), 2.69 (t, 4H), 2.53 (br, 6H), 2.12 (t, 4H), 2.28 (t, 4H), 1.66 - 1.26 (m, 62H), 0.89 (t, 6H)

### Preparation Example 4

### 4-1. The compound of the following formula D was prepared according to the synthesis scheme shown in Figure 4.

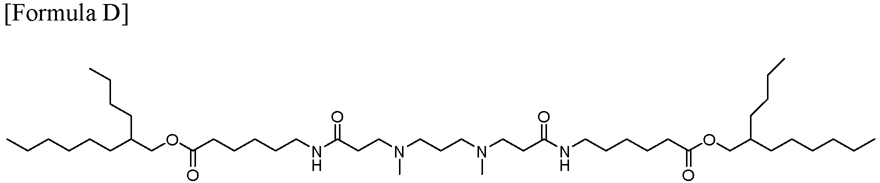

### 4-2. Synthesis of 2-butyloctyl 6-acrylamidohexanoate

In a 500 mL 3-neck RBF, 6-aminohexanoic acid (5.03 g, 38.32 mmol, 1.20 eq), 2-butyl-1-n-octanol (5.95 g, 31.93 mmol, 1.00 eq), p-toluenesulfonic acid monohydrate (10.93 g, 57.48 mmol, 1.80 eq), and cyclohexane (200 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-butyloctyl 6-aminohexanoate with low purity. Without further purification, previously obtained 2-butyloctyl 6-aminohexanoate, methylene chloride (170 mL), and triethylamine (7.11 g, 70.28 mmol, 2.20 eq) were added to a 250 mL 3-neck RBF, cooled to 0°C, and acryloyl chloride (3.18 g, 35.12 mmol, 1.10 eq) was added dropwise. The temperature of the reactor was raised to room temperature (20-25°C) and stirring was performed. After 18 hours, the mixture in the reactor was extracted with HCl aqueous solution, and the organic layer was dried over sodium sulfate and filtered. The filtrate was concentrated in vacuo and purified using a silica column with ethyl acetate (EtOAc):hexane (1:2) to obtain 2-butyloctyl 6-acrylamidohexanoate (5.0 g, yield: 44%).
¹H-NMR (400 MHz, CDCl₃) *δ* 6.47 (dd, 1H), 6.09-6.15 (m, 1H), 5.63 (dd, 2H), 3.97 (d, 2H), 3.34 (q, 2H), 2.32 (t, 2H), 1.54-1.68 (m, 6H), 1.27-1.40 (m, 20H), 0.87-0.91 (m, 6H)

### 4-3. Synthesis of the compound of formula D

In a 100 mL 3-neck RBF, 2-butyloctyl 6-acrylamidohexanoate (1000.00 mg, 2.83 mmol, 2.60 eq), N,N'-dimethyl-1,3-propanediamine (111.16 mg, 1.09 mmol, 1.00 eq), and n-BuOH (11 mL) were added, and then stirring and reflux were performed. After 24 hours, the mixture was concentrated in vacuo at 80°C, and purified using a silica column with methylene chloride: methanol: ammonium hydroxide (15:1:0.1) to obtain the compound of formula D (712.60 mg, yield: 81%).

¹H-NMR (400 MHz, CDCl₃) δ 7.87 (s, 2H), 3.96 (d, 4H), 3.22 (q, 4H), 2.61 (t, 4H), 2.41 (t, 4H), 2.36 (t, 4H), 2.30 (t, 4H), 2.25 (s, 6H), 1.61-1.70 (m, 8H), 1.48-1.54 (m, 4H), 1.25-1.39 (m, 37H), 0.90 (t, 3H)

### Preparation Example 5

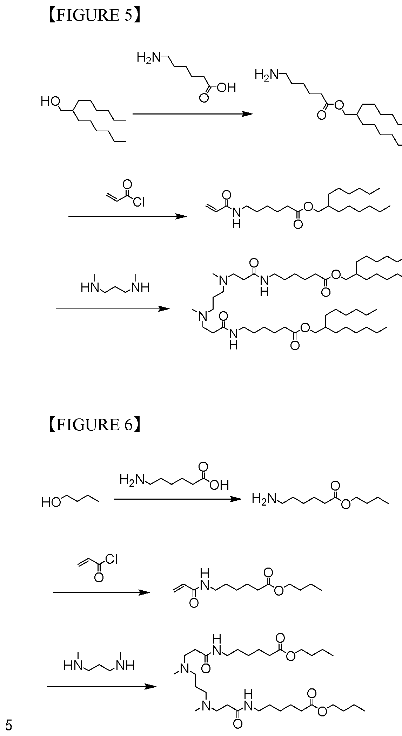

### 5-1. The compound of the following formula E was prepared according to the synthesis scheme shown in Figure 5.

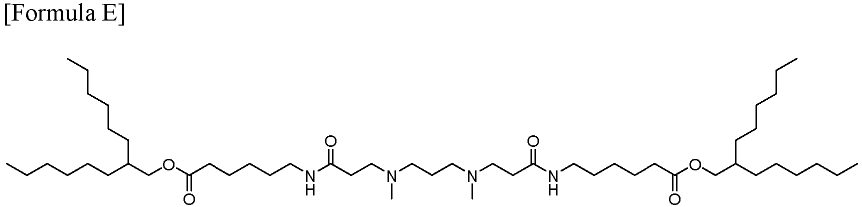

### 5-2. Synthesis of 2-hexyloctyl 6-acrylamidohexanoate

In a 500 mL 3-neck RBF, 6-aminohexanoic acid (1.84 g, 13.99 mmol, 1.20 eq), 2-hexyl-1-n-octanol (2.50 g, 11.66 mmol, 1.00 eq), p-toluenesulfonic acid monohydrate (3.99 g, 20.99 mmol, 1.80 eq), and cyclohexane (120 mL) were added, and a Dean-Stark trap and a condenser were installed and then stirring and reflux were performed. After 24 hours, the mixture was cooled to room temperature, concentrated in vacuo, extracted with methylene chloride and 3% aqueous sodium hydroxide solution, and the methylene chloride layer was dried over sodium sulfate and filtered. The filtrate was concentrated in vacuo to obtain 2-hexyloctyl 6-aminohexanoate with low purity. Without further purification, the previously obtained 2-hexyloctyl 6-aminohexanoate, methylene chloride (60 mL), triethylamine (2.60 g, 25.65 mmol, 2.20 eq), and methylene chloride (60 mL) were added to a 250 mL 3-neck RBF and cooled to 0°C, and acryloyl chloride (1.16 g, 12.83 mmol, 1.10 eq) was added dropwise. The temperature of the reactor was raised to room temperature (20-25°C) and stirring was performed. After 18 hours, the mixture in the reactor was extracted with HCl aqueous solution, and the organic layer was dried over sodium sulfate and filtered. The filtrate was concentrated in vacuo and purified using a silica column with ethyl acetate (EtOAc):hexane (1:2) to obtain 2-hexyloctyl 6-acrylamidohexanoate (3.63 g, yield: 82%).
¹H-NMR (400 MHz, CDCl₃) *δ* 6.27 (dd, 1H), 6.05-6.11 (m, 1H), 5.63 (dd, 2H), 3.97 (d, 2H), 3.34 (q, 2H), 2.32 (t, 2H), 1.41-1.68 (m, 6H), 1.21-1.41 (m, 20H), 0.85-0.90 (m, 6H)

### 5-3. Synthesis of the compound of formula E

In a 100 mL 3-neck RBF, 2-hexyloctyl 6-acrylamidohexanoate (660.27 mg, 1.73 mmol, 2.60 eq), N,N'-dimethyl-1,3-propanediamine (68.00 mg, 0.67 mmol, 1.00 eq), and n-BuOH (7 mL) were added, and then stirring and reflux were performed. After 24 hours, the mixture was concentrated in vacuo at 80°C and purified using a silica column with methylene chloride: methanol: ammonium hydroxide (15:1:0.1) to obtain the compound of formula E (375.60 mg, yield: 65%).
¹H-NMR (400 MHz, CDCl₃) δ 7.85 (s, 2H), 3.96 (d, 4H), 3.22 (q, 4H), 2.62 (t, 4H), 2.42 (t, 4H), 2.36 (t, 4H), 2.30 (t, 4H), 2.25 (s, 6H), 1.61-1.71 (m, 8H), 1.48-1.54 (m, 4H), 1.20-1.40 (m, 45H), 0.90 (t, 12H)

### Preparation Example 6

### 6-1. The compound of the following formula F was prepared according to the synthesis scheme shown in Figure 6.

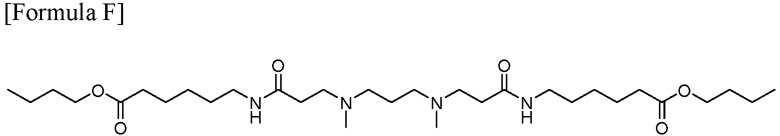

### 6-2. Synthesis of butyl 6-acrylamidohexanoate

In a 500 mL 3-neck RBF, 6-aminohexanoic acid (6.37 g, 48.57 mmol, 1.20 eq), butan-1-ol (3.00 g, 40.47 mmol, 1.00 eq), p-toluenesulfonic acid monohydrate (15.40 g, 80.95 mmol, 2.00 eq), and cyclohexane (200 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain butyl 6-aminohexanoate with low purity. Without further purification, previously obtained butyl 6-aminohexanoate, methylene chloride (200 mL), and triethylamine (9.01 g, 89.04 mmol, 2.20 eq) were added to a 500 mL 3-neck RBF and cooled to 0°C, and acryloyl chloride (4.03 g, 44.52 mmol, 1.10 eq) was added dropwise. The temperature of the reactor was raised to room temperature (20-25 °C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:1) to obtain butyl 6-acrylamidohexanoate (3.58 g, yield: 37%).
¹H-NMR (400 MHz, CDCl₃) *δ* 6.27 (dd, 1H), 6.01-6.11 (m, 1H), 5.62-5.67 (m, 2H), 4.07 (t, 2H), 3.34 (q, 2H), 2.31 (t, 2H), 1.41-1.62 (m, 6H), 1.34-1.39 (m, 4H), 0.93 (t, 3H)

### 6-3. Synthesis of the compound of formula F

In a 100 mL 3-neck RBF, butyl 6-acrylamidohexanoate (700.00 mg, 2.90 mmol, 2.60 eq), N,N'-dimethyl-1,3-propanediamine (113.99 mg, 1.12 mmol, 1.00 eq), and n-BuOH (11 mL) were added, and then stirring and reflux were performed. After 24 hours, the mixture was concentrated in vacuo at 80°C and purified using a silica column with methylene chloride:methanol:ammonium hydroxide (15:1:0.1) to obtain the compound of formula F (479.00 mg, yield: 73%).
¹H-NMR (400 MHz, CDCl₃) *δ* 7.89 (s, 2H), 4.06 (t, 4H), 3.22 (q, 4H), 2.61 (t, 4H), 2.46 (t, 4H), 2.42 (t, 4H), 2.36 (t, 4H), 2.31 (s, 6H), 1.60-1.70 (m, 10H), 1.41-1.60 (m, 4), 1.32-1.39 (m, 8H), 0.90 (t, 6H)

### Preparation Example 7

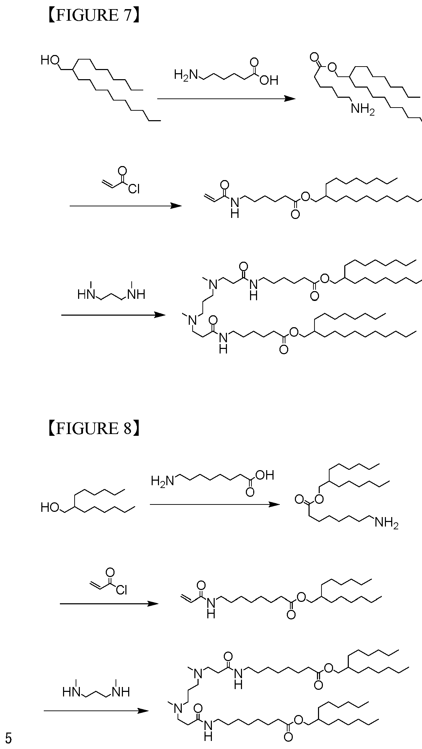

### 7-1. The compound of the following formula G was prepared according to the synthesis scheme shown in Figure 7.

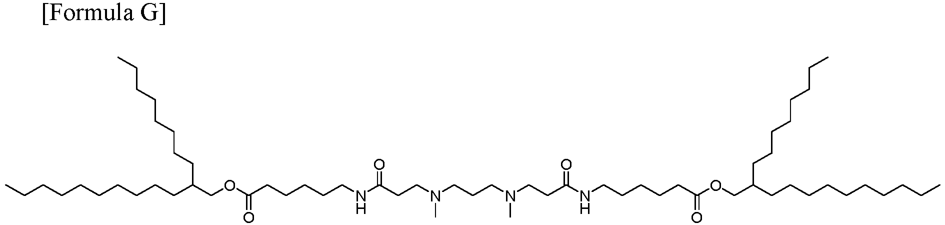

### 7-2. Synthesis of 2-octyldodecyl 6-acrylamidohexanoate

In a 250 mL 3-neck RBF, 6-aminohexanoic acid (1.05 g, 8.04 mmol, 1.20 eq), 2-octyldodecan-1-ol (2.00 g, 6.70 mmol, 1.00 eq), p-toluenesulfonic acid monohydrate (2.29 g, 12.06 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-octyldodecyl 6-aminohexanoate with low purity. Without further purification, previously obtained 2-octyldodecyl 6-aminohexanoate, methylene chloride (33 mL), and triethylamine (1.49 g, 14.74 mmol, 2.20 eq) were added to a 100 mL 3-neck RBF and cooled to 0°C, and acryloyl chloride (0.67 g, 7.37 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:2) to obtain 2-octyldodecyl 6-acrylamidohexanoate (1928.20 mg, yield: 62%).
¹H-NMR (400 MHz, CDCl₃) *δ* 6.27 (dd, 1H), 6.05-6.10 (m, 1H), 5.59-5.64 (m, 2H), 3.97 (d, 2H), 3.34 (q, 2H), 2.32 (t, 2H), 1.54-1.68 (m, 4H), 1.26-1.41 (m, 36H), 0.89 (t, 6H)

### 7-3. Synthesis of the compound of formula G

In a 100 mL 3-neck RBF, 2-octyldodecyl 6-acrylamidohexanoate (592.57 mg, 1.27 mmol, 2.60 eq), N,N'-dimethyl-1,3-propanediamine (50.00 mg, 0.49 mmol, 1.00 eq), and n-BuOH (5 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of formula G (346.8 mg, yield: 69%).
¹H-NMR (400 MHz, CDCl₃) *δ* 7.83 (s, 2H), 3.96 (d, 4H), 3.22 (q, 4H), 2.62 (t, 4H), 2.42 (t, 4H), 2.36 (t, 4H), 2.30 (t, 4H), 2.25 (s, 6H), 1.61-1.69 (m, 8H), 1.48-1.54 (m, 4H), 1.25-1.39 (m, 69H), 0.90 (t, 3H)

### Preparation Example 8

### 8-1. The compound of the following formula H was prepared according to the synthesis scheme shown in Figure 8.

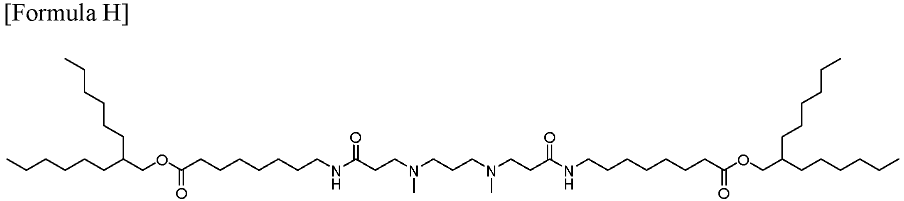

### 8-2. Synthesis of 2-hexyloctyl 8-acrylamidooctanoate

In a 250 mL 3-neck RBF, 8-aminoctanoic acid (1.78 g, 11.19 mmol, 1.20 eq), 2-hexyloctan-1-ol (2.00 g, 9.33 mmol, 1.00 eq), p-toluenesulfonic acid monohydrate (3.19 g, 16.79 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-hexyloctyl 8-aminooctanoate with low purity. Without further purification, previously obtained 2-hexyloctyl 8-aminooctanoate, methylene chloride (100 mL), and triethylamine (2.08 g, 20.52 mmol, 2.20 eq) were added to a 250 mL 3-neck RBF and cooled to 0°C, and acryloyl chloride (0.93 g, 10.26 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:1) to obtain 2-hexyloctyl 8-acrylamidooctanoate (2838.70 mg, yield: 74%).
¹H-NMR (400 MHz, CDCl₃) *δ* 6.27 (dd, 1H), 6.05-6.10 (m, 1H), 5.55-5.64 (m, 2H), 3.97 (d, 2H), 3.32 (q, 2H), 2.29 (t, 2H), 1.51-1.65 (m, 4H), 1.27-1.35 (m, 27H), 0.85-0.90 (m, 6H)

### 8-3. Synthesis of the compound of formula H

In a 100 mL 3-neck RBF, 2-hexyloctyl 8-acrylamidooctanoate (703.42 mg, 0.76 mmol, 2.60 eq), N,N'-dimethyl-1,3-propanediamine (30.00 mg, 0.29 mmol, 1.00 eq), and n-BuOH (5 mL) were added, and then stirring and reflux were performed. After 24 hours, the mixture was concentrated in vacuo at 80°C and purified using a silica column with methylene chloride: methanol: ammonium hydroxide (10:1:0.1) to obtain the compound of formula H (218.90 mg, yield: 81%).
¹H-NMR (400 MHz, CDCl₃) δ 7.78 (s, 2H), 3.96 (d, 4H), 3.21 (q, 4H), 2.64 (t, 4H), 2.44 (t, 4H), 2.38 (t, 4H), 2.28-2.32 (m, 4H), 2.26 (s, 6H), 1.66-1.72 (m, 2H), 1.40-1.63 (m, 10H), 1.20-1.40 (m, 54H), 0.90 (t, 12H)

### Preparation Example 9

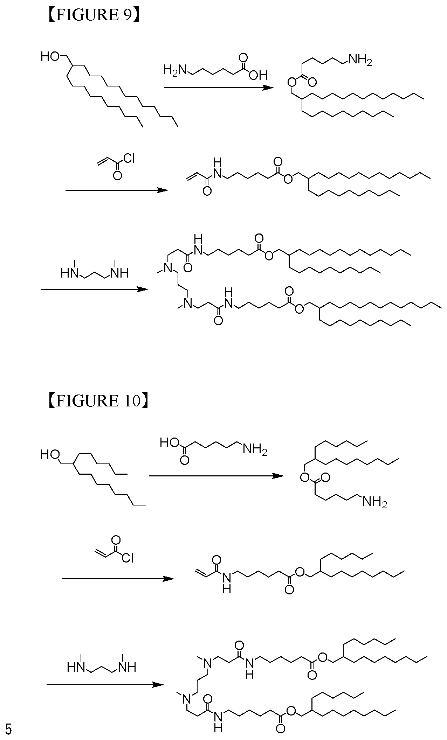

### 9-1. The compound of the following formula I was prepared according to the synthesis scheme shown in Figure 9.

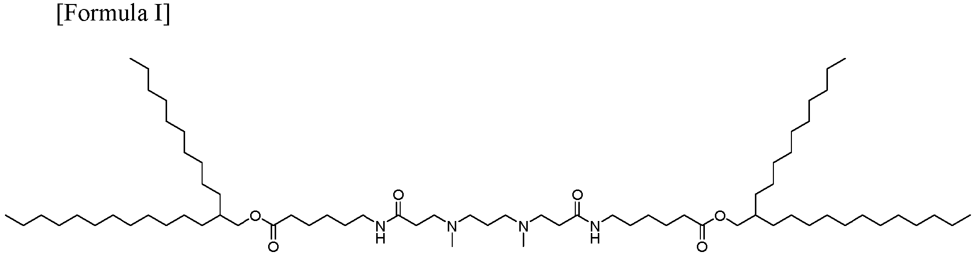

### 9-2. Synthesis of 2-decyltetradecyl 6-acrylamidohexanoate

In a 250 mL 3-neck RBF, 6-aminohexanoic acid (887.70 mg, 6.77 mmol, 1.20 eq), 2-decyltetradecan-1-ol (2.00 g, 5.64 mmol, 1.00 eq), p-toluenesulfonic acid monohydrate (1.93 g, 10.15 mmol, 1.80 eq), and cyclohexane (100 mL) were added, and a Dean-Stark trap and a condenser were installed and then stirring and reflux were performed. After 24 hours, the mixture was cooled to room temperature, concentrated in vacuo, extracted with methylene chloride and 3% sodium hydroxide aqueous solution, and the methylene chloride layer was dried over sodium sulfate and filtered. The filtrate was concentrated in vacuo to obtain 2-decyltetradecyl 6-aminohexanoate with low purity. Without further purification, previously obtained 2-decyltetradecyl 6-aminohexanoate, methylene chloride (100 mL), and triethylamine (1.26 g, 12.41 mmol, 2.20 eq) were added to a 250 mL 3-neck RBF and cooled to 0°C, and acryloyl chloride (561.44 mg, 6.20 mmol, 1.10 eq) was added dropwise. The temperature of the reactor was raised to room temperature (20-25°C) and stirring was performed. After 18 hours, the mixture in the reactor was extracted with aqueous HCl solution, and the organic layer was dried over sodium sulfate and filtered. The filtrate was concentrated in vacuo and purified using a silica column with EtOAc:hexane (1:2) to obtain 2-decyltetradecyl 6-acrylamidohexanoate (1.29 g, yield: 44%).
¹H-NMR (400 MHz, CDCl₃) *δ* 6.27 (dd, 1H), 6.05-6.10 (m, 1H), 5.62-5.64 (m, 2H), 3.97 (d, 2H), 3.34 (q, 2H) 2.31 (t, 2H), 1.54-1.68 (m, 5H), 1.26-1.41 (m, 44H), 0.88-0.89 (m, 6H)

### 9-3. Synthesis of the compound of formula I

In a 100 mL 3-neck RBF, 2-decyltetradecyl 6-acrylamidohexanoate (306.44 mg, 0.59 mmol, 2.40 eq), N,N'-dimethyl-1,3-propanediamine (25.00 mg, 0.24 mmol, 1.00 eq), and n-BuOH (2.5 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula I (209.2 mg, yield: 75%).
¹H-NMR (400 MHz, CDCl3) δ 7.86 (s, 2H), 3.96 (d, 4H), 3.22 (q, 4H), 2.60 (t, 4H), 2.41 (t, 4H), 2.35 (t, 4H), 2.30 (t, 4H), 2.24 (s, 6H), 1.61-1.69 (m, 11H), 1.48-1.53 (m, 4H), 1.20-1.40 (m, 87H), 0.9 (t, 12H)

### Preparation Example 10

### 10-1. The compound of the following formula J was prepared according to the synthesis scheme shown in Figure 10.

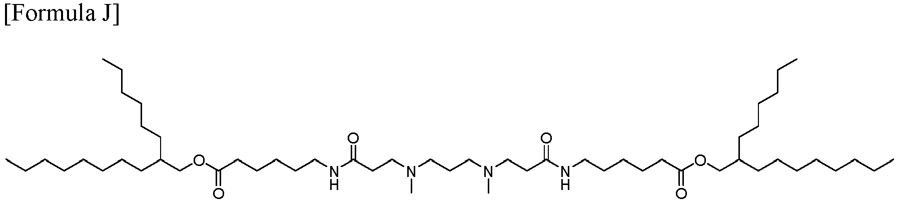

### 10-2. Synthesis of the compound of formula J

In a 100 mL 3-neck RBF, 2-hexyldecyl 6-acrylamidohexanoate (2000.00 mg, 4.88 mmol, 3.00 eq) synthesized in Example 2-2, N,N'-dimethyl-1,3-propanediamine (170.00 mg, 1.63 mmol, 1.00 eq), and n-BuOH (20 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of chemical formula J (934.00 mg, yield: 62%).
¹H-NMR (400 MHz, CDCl₃) δ 7.88 (s, 2H), 3.96 (d, 4H), 3.22 (q, 4H), 2.60 (t, 4H), 2.40 (t, 4H), 2.35 (t, 4H), 2.30 (t, 4H), 2.27 (s, 6H), 1.61-1.70 (m, 8H), 1.40-1.54 (m, 4H), 1.20-1.40 (m, 52H), 0.90 (t, 12H)

### Preparation Example 11

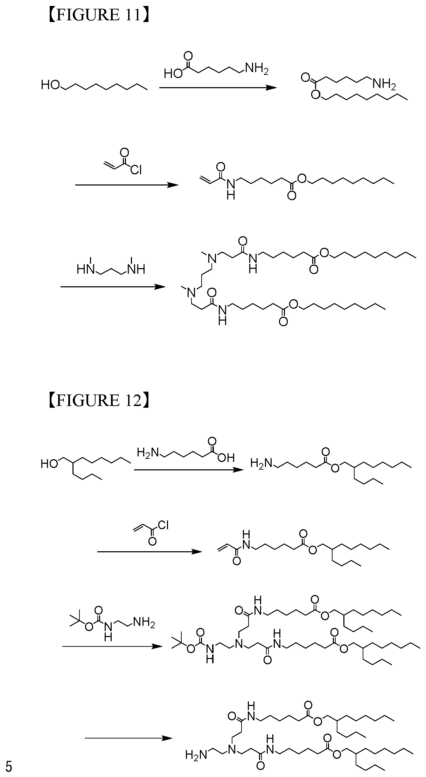

### 11-1. The compound of the following formula K was prepared according to the synthesis scheme shown in Figure 11.

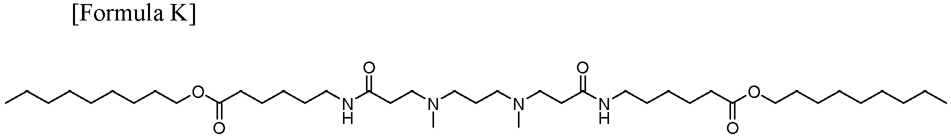

### 11-2. Synthesis of nonyl 6-acrylamidohexanoate

In a 250 mL 3-neck RBF, 6-aminohexanoic acid (10 g, 76.23 mmol, 1.1 eq), nonan-1-ol (21.58 g, 69.30 mmol, 1 eq), p-toluenesulfonic acid monohydrate (p-TsOH) (15.82 g, 83.16 mmol, 1.2 eq), and cyclohexane (120 mL) were added, and a Dean-Stark trap and a condenser were installed and then stirring and reflux were performed. After 24 hours, the mixture was concentrated in vacuo, extracted with methylene chloride and 3% aqueous sodium hydroxide solution, and the methylene chloride layer was dried over sodium sulfate and filtered. The filtrate was concentrated in vacuo to obtain nonyl 6-aminohexanoate with low purity. Without further purification, previously obtained nonyl 6-aminohexanoate, methylene chloride (100 mL), and triethylamine (15.43 g, 152.46 mmol, 2.2 eq) were added to a 250 mL 3-neck RBF and cooled to 0°C, and acryloyl chloride (6.90 g, 76.23 mmol, 1.1 eq) was added dropwise. The temperature of the reactor was raised to room temperature (20-25°C) and stirring was performed. After 4 hours, the mixture in the reactor was extracted with a saturated aqueous sodium bicarbonate solution, and the methylene chloride layer was dried over sodium sulfate and filtered. The filtrate was concentrated in vacuo and purified using a silica column with EtOAc:hexane (1:1) to obtain nonyl 6-acrylamidohexanoate (15.66 g, yield: 66%).
¹H-NMR (400 MHz, CDCl₃) *δ* 6.28 (d, 1H), 6.11 (m, 1H), 5.64 (d, 2H), 4.07 (t, 2H), 3.63 (q, 2H), 2.32 (t, 2H), 1.68 - 1.26 (m, 20H), 0.90 (t, 3H)

### 11-3. Synthesis of the compound of formula K

In a 100 mL 3-neck RBF, nonyl 6-acrylamidohexanoate (1000.00 mg, 3.21 mmol, 2.60 eq), N,N'-dimethyl-1,3-propanediamine (126.18 mg, 1.23 mmol, 1.00 eq), and n-BuOH (10 mL) were added, and then stirring and reflux were performed. After 24 hours, the mixture was concentrated in vacuo at 80°C and purified using a silica column with methylene chloride:methanol:ammonium hydroxide (7:1:0.1) to obtain the compound of formula K (770.10 mg, yield: 86%).
¹H-NMR (400 MHz, CDCl₃) *δ* 7.87 (s, 2H), 4.05 (t, 4H), 3.22 (q, 4H), 2.62 (t, 4H), 2.42 (t, 4H), 2.36 (t, 2H), 2.30 (t, 4H), 2.25 (s, 6H), 1.61-1.69 (m, 10H), 1.40-1.60 (m, 4H), 1.27-1.38 (m, 28H), 0.87 (t, 6H)

### Preparation Example 12

### 12-1. The compound of the following formula L was prepared according to the synthesis scheme shown in Figure 12.

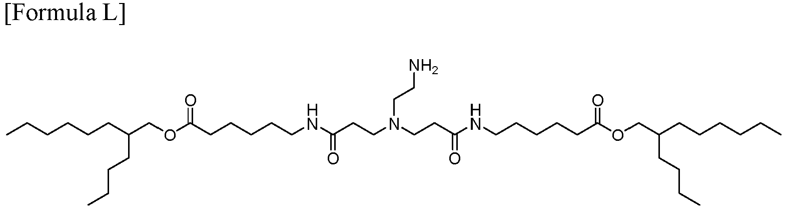

### 12-2. Synthesis of the compound of formula L

In a 100 mL 3-neck RBF, 2-butyloctyl 6-acrylamidohexanoate (850.65 mg, 2.43 mmol, 2.60 eq) synthesized in Example 4-2, tert-butyl N-(2-aminoethyl)carbamate (150.00 mg, 0.94 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-butyloctyl 8-(3-((6-((2-butyloctyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula L was conducted without further purification process. In a 25 mL 1-neck RBF, 2-butyloctyl 8-(3-((6-((2-butyloctyl)oxy)-6-oxohexyl)amino)-3-oxopropyl)-2,2-dimethyl-4,11-dioxo-3-oxa-5,8,12-triazaoctadecan-18-oate (143.20 mg, 0.16 mmol) and methylene chloride (2 mL) were added, and trifluoroacetic acid (0.2 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (7:1:0.1) to obtain the compound of formula L (85.1 mg, 67%).
¹H-NMR (400 MHz, CDCl₃) δ 6.38 (t, 2H), 3.96 (d, 4H), 3.18 (q, 4H), 2.93 (t, 2H), 2.67 (t, 4H), 2.58 (t, 2H), 2.29-2.36 (m, 8H), 1.61-1.66 (m, 6H), 1.48-1.60 (m, 4H), 1.27-1.38 (m, 38H), 0.90-0.92 (m, 12H)

### Preparation Example 13

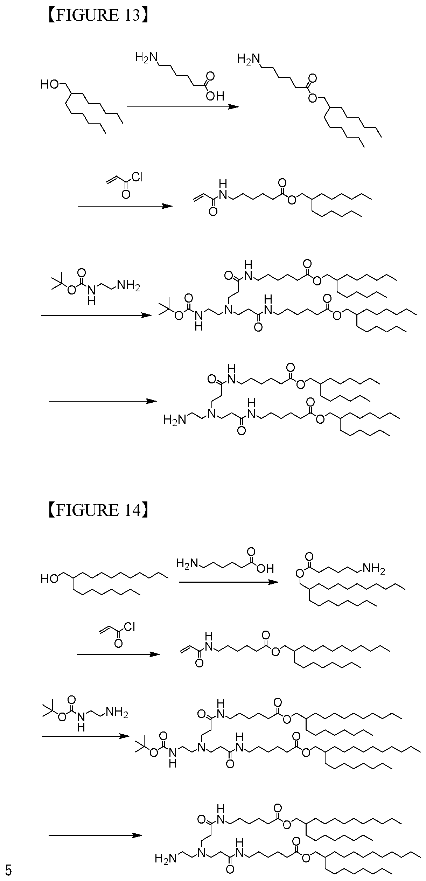

### 13-1. The compound of the following formula M was prepared according to the synthesis scheme shown in Figure 13.

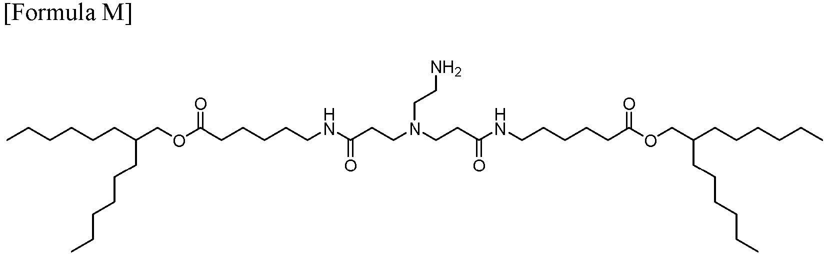

### 13-2. Synthesis of the compound of formula M

In a 100 mL 3-neck RBF, 2-hexyloctyl 6-acrylamidohexanoate (1429.07 mg, 3.74 mmol, 2.40 eq) synthesized in Example 5-2, tert-butyl N-(2-aminoethyl)carbamate (250.00 mg, 1.56 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-hexyloctyl 8-(3-((6-((2-hexyloctyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula M was conducted without further purification process. In a 25 mL 1-neck RBF, 2-hexyloctyl 8-(3-((6-((2-hexyloctyl)oxy)-6-oxohexyl)amino)-3-oxopropyl)-2,2-dimethyl-4,11-dioxo-3-oxa-5 ,8,12-triazaoctadecan-18-oate and methylene chloride (3 mL) were added, and trifluoroacetic acid (0.3 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (8:1:0.1) to obtain the compound of formula M (116.6 mg, two steps 9.1%).
¹H-NMR (400 MHz, CDCl₃) δ 6.94 (t, 2H), 3.96 (d, 4H), 3.12-3.20 (m, 6H), 2.73 (t, 2H), 2.62 (t, 4H), 2.29-2.37 (m, 8H), 1.59-1.65 (m, 6H), 1.47-1.52 (m, 4H), 1.20-1.39 (m, 46H), 0.90 (t, 12H)

### Preparation Example 14

### 14-1. The compound of the following formula N was prepared according to the synthesis scheme shown in Figure 14.

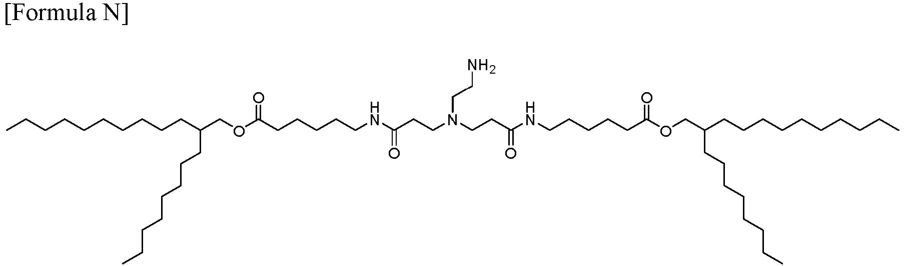

### 14-2. Synthesis of the compound of formula N

In a 100 mL 3-neck RBF, 2-octyldodecyl 6-acrylamidohexanoate (1200.00 mg, 2.58 mmol, 2.40 eq) synthesized in Example 7-2, tert-butyl N-(2-aminoethyl)carbamate (171.99 mg, 1.07 mmol, 1.00 eq), and n-BuOH (10 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-octyldodecyl 2,2-dimethyl-8-(3-((6-((2-octyldodecyl)oxy)-6-oxohexyl)amino)-3-oxopropyl)-4,11-dioxo-3-oxa-5,8,12-triazaoctadecan-18-oate with low purity. Subsequently, the synthesis of the compound of formula N was conducted without further purification process. In a 100 mL 1-neck RBF, 2-octyldodecyl 2,2-dimethyl-8-(3-((6-((2-octyldodecyl)oxy)-6-oxohexyl)amino)-3-oxopropyl)-4,11-dioxo-3-oxa-5,8,12-triazaoctadecan-18-oate and methylene chloride (10 mL) were added, and trifluoroacetic acid (1.0 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (7:1:0.1) to obtain the compound of formula N (137.8 mg, two steps 12.9%).
¹H-NMR (400 MHz, CDCl₃) δ 6.86 (t, 2H), 3.96 (d, 4H), 3.17 (q, 4H), 2.98 (t, 2H), 2.61-2.67 (m, 6H), 2.29-2.37 (m, 8H), 1.60-1.66 (m, 7H), 1.20-1.38 (m, 72H), 0.90-0.95 (m, 12H)

### Preparation Example 15

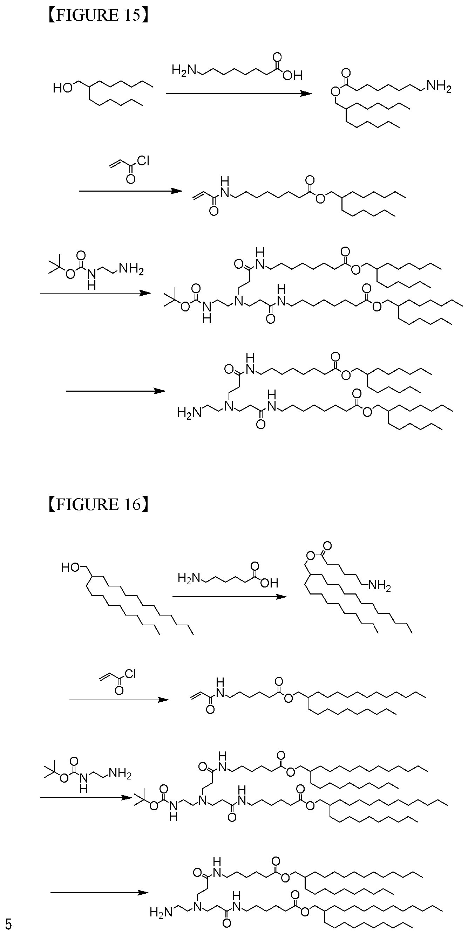

### 15-1. The compound of the following formula O was prepared according to the synthesis scheme shown in Figure 15.

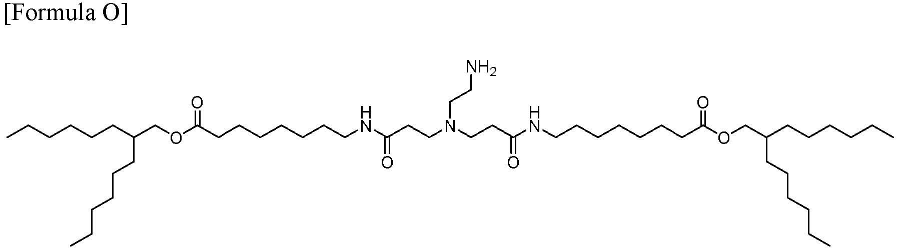

### 15-2. Synthesis of the compound of formula O

In a 100 mL 3-neck RBF, 2-hexyloctyl 8-acrylamidooctanoate (1130.14 mg, 2.76 mmol, 2.60 eq) synthesized in Example 8-2, tert-butyl N-(2-aminoethyl)carbamate (170.00 mg, 1.06 mmol, 1.00 eq), and n-BuOH (11 mL) were added, and then stirring and reflux were performed. After 48 hours, the mixture was concentrated in vacuo at 80°C and purified using a silica column with methylene chloride:methanol:ammonium hydroxide (15:1:0.1) to obtain 2-hexyloctyl 8-(3-((8-((2-hexyloctyl)oxy)-8-oxooctyl)amino)-3-oxopropyl)-2,2-dimethyl-4,11-dioxo-3-oxa-5,8,12-triazaicosan-20-oate with low purity. Subsequently, the synthesis of the compound of formula O was conducted without further purification process. In a 25 mL 1-neck RBF, 2-hexyloctyl 8-(3-((8-((2-hexyloctyl)oxy)-8-oxooctyl)amino)-3-oxopropyl)-2,2-dimethyl-4,11-dioxo-3-oxa-5 ,8,12-triazicosane-20-oate and methylene chloride (4.4 mL) were added, and trifluoroacetic acid (0.4 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (10:1:0.1) to obtain the compound of formula O (225.5 mg, two steps 24.2%).
¹H-NMR (400 MHz, CDCl₃) δ 6.81 (t, 2H), 3.96 (d, 4H), 3.15 (q, 4H), 3.03 (t, 2H), 2.63-2.66 (m, 6H), 2.35 (t, 4H), 2.29 (t, 4H), 1.59-1.62 (m, 6H), 1.46-1.50 (m, 4H), 1.27-1.39 (s, 55H), 0.87-0.92 (m, 12H)

### Preparation Example 16

### 16-1. The compound of the following formula P was prepared according to the synthesis scheme shown in Figure 16.

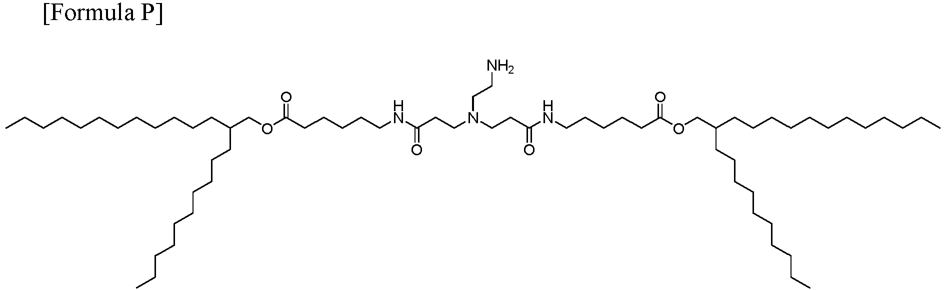

### 16-2. Synthesis of the compound of formula P

In a 100 mL 3-neck RBF, 2-decyltetradecyl 6-acrylamidohexanoate (891.15 mg, 1.71 mmol, 2.40 eq) synthesized in Example 9-2, tert-butyl N-(2-aminoethyl)carbamate (114.00 mg, 0.71 mmol, 1.00 eq), and n-BuOH (6.2 mL) were added, and then stirring and reflux were performed. After 48 hours, the mixture was concentrated in vacuo at 80°C and purified using a silica column with methylene chloride:methanol:ammonium hydroxide (20:1:0.1) to obtain 2-decyltetradecyl 8-(3-((6-((2-decyltetradecyl)oxy)-6-oxohexyl)amino)-3-oxopropyl)-2,2-dimethyl-4,11-dioxo-3-oxa-5,8,12-triazaoctadecan-18-oate with low purity. Subsequently, the synthesis of the compound of formula P was conducted without further purification process. In a 25 mL 1-neck RBF, 2-decyltetradecyl 8-(3-((6-((2-decyltetradecyl)oxy)-6-oxohexyl)amino)-3-oxopropyl)-2,2-dimethyl-4,11-dioxo-3-oxa-5,8,12-triazaoctadecan-18-oate and methylene chloride (6.0 mL) were added, and trifluoroacetic acid (0.6 mL) was added dropwise. After stirring at room temperature for 24 hours, the mixture in the reactor was extracted with a saturated aqueous solution of sodium bicarbonate, and the organic layer was dried over sodium sulfate and filtered. The filtrate was concentrated in vacuo and purified using a silica column with methylene chloride:methanol:ammonium hydroxide (15:1:0.1) to obtain the compound of chemical formula P (157.9 mg, two steps 20.1%).
¹H-NMR (400 MHz, CDCl₃) δ 6.98 (t, 2H), 3.96 (d, 4H), 3,16 (q, 4H), 3.01 (t, 2H), 2.65 (t, 6H), 2.36 (t, 4H), 2.30 (t, 4H), 1.60-1.66 (m, 7H), 1.48-1.54 (m, 5H), 1.20-1.40 (m, 83H), 0.90-0.95 (m, 12H)

### Preparation Example 17

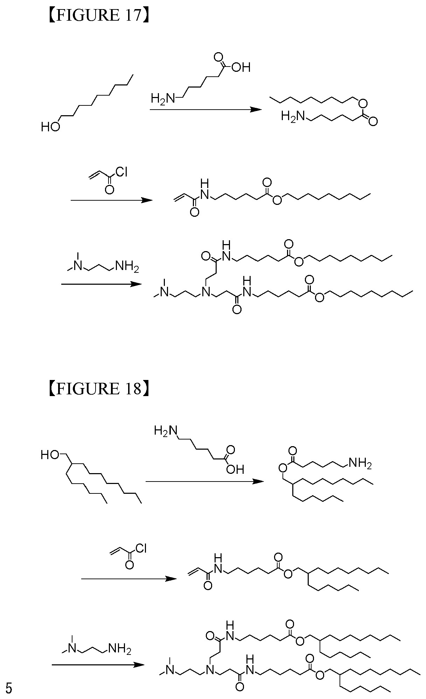

### 17-1. The compound of the following formula Q was prepared according to the synthesis scheme shown in Figure 17.

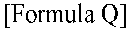

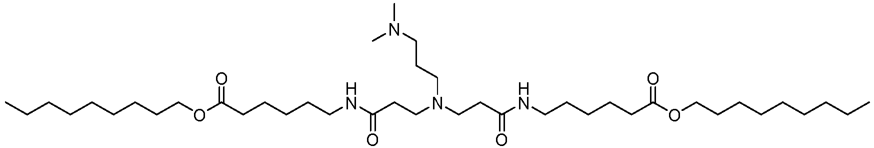

### 17-2. Synthesis of the compound of formula Q

In a 100 mL 3-neck RBF, nonyl 6-acrylamidohexanoate (3000.00 mg, 9.63 mmol, 2.60 eq) synthesized in Example 11-2, N,N-dimethylpropanediamine (378.53 mg, 1.00 mmol, 1.00 eq), and n-BuOH (18 mL) were added, and then stirring and reflux were performed. After 24 hours, the mixture was concentrated in vacuo at 80°C and purified using a silica column with methylene chloride:methanol:ammonium hydroxide (7:1:0.1) to obtain the compound of formula Q (1257.6 mg, yield: 47%).
¹H-NMR (400 MHz, CDCl₃) δ 4.05 (t, 4H), 3.22 (q, 4H), 2.73 (t, 4H), 2.50 (t, 2H), 2.25-2.47 (m, 10H), 2.23 (s, 6H), 1.60-1.67 (m, 10H), 1.48-1.58 (m, 4H), 1.27-1.38 (m, 28H), 0.88 (t, 6H)

### Preparation Example 18

### 18-1. The compound of the following formula R was prepared according to the synthesis scheme shown in Figure 18.

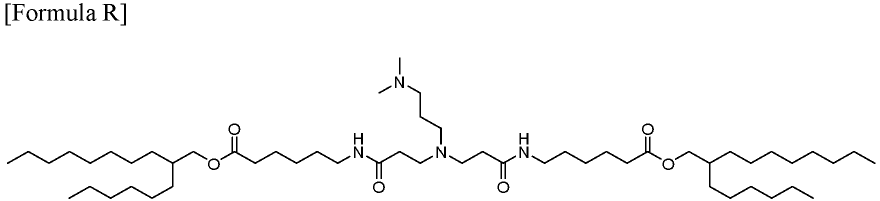

### 18-2. Synthesis of the compound of formula R

In a 100 mL 3-neck RBF, 2-hexyldecyl 6-acrylamidohexanoate (1500.00 mg, 3.67 mmol, 2.60 eq) synthesized in Example 2-2, N,N-dimethylpropanediamine (143.90 mg, 1.41 mmol, 1.00 eq), and n-BuOH (7 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula R (476.10 mg, yield: 37%).
¹H-NMR (400 MHz, CDCl₃) δ 6.88 (t, 2H), 3.97 (d, 4H), 3.21 (q, 4H), 2.72 (t, 4H), 2.49 (t, 2H), 2.29-2.35 (m, 10H), 2.22 (s, 6H), 1.61-1.67 (m, 9H), 1.49-1.55 (m, 4H), 1.27-1.40 (m, 50H), 0.88 (t, 12H)

### Preparation Example 19

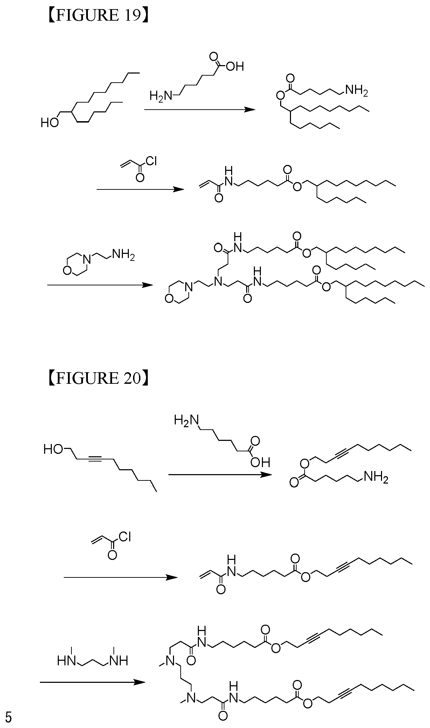

### 19-1. The compound of the following formula S was prepared according to the synthesis scheme shown in Figure 19.

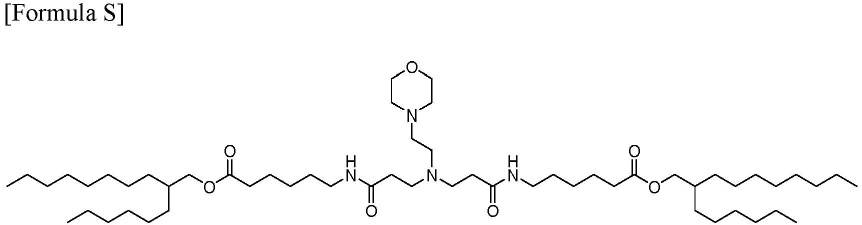

### 19-2. Synthesis of the compound of formula S

In a 100 mL 3-neck RBF, 2-hexyldecyl 6-acrylamidohexanoate (2000.00 mg, 4.88 mmol, 3.00 eq) synthesized in Example 2-2, 2-morpholinoethan-1-ol (210.00 mg, 1.63 mmol, 1.00 eq), and n-BuOH (20 mL) were added, and then stirring and reflux were performed. After 48 hours, the mixture was concentrated in vacuo at 80°C, and purified using a silica column with methylene chloride:methanol:ammonium hydroxide (15:1:0.1) to obtain the compound of chemical formula S (596.00 mg, yield: 39%).
¹H-NMR (400 MHz, CDCl₃) δ 6.82 (t, 2H), 3.96 (d, 4H), 3.69 (t, 4H), 3.22 (q, 4H), 2.76 (t, 3H), 2.58 (t, 2H), 2.43-2.49 (m, 6H), 2.29-2.34 (m, 7H), 1.61-1.67 (m, 6H), 1.38-1.54 (m, 4H), 1.27-1.38 (m, 52H), 0.88 (t, 12H)

### Preparation Example 20

### 20-1. The compound of the following formula T was prepared according to the synthesis scheme shown in Figure 20.

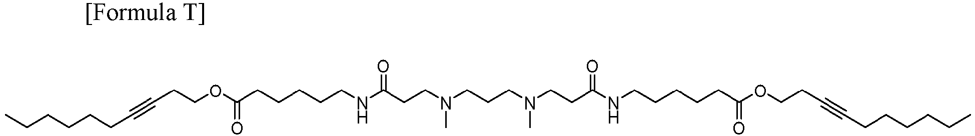

### 20-2. Synthesis of dec-3-yn-1-yl 6-acrylamidohexanoate

In a 500 mL 3-neck RBF, 6-aminohexanoic acid (3.06 g, 23.34 mmol, 1.20 eq), dec-3-yn-1-ol (3.00 g, 19.45 mmol, 1.00 eq), p-toluenesulfonic acid monohydrate (5.55 g, 29.17 mmol, 1.50 eq), and cyclohexane (130 mL) were added, and a Dean-Stark trap and a condenser were installed and then stirring and reflux were performed. After 24 hours, the mixture was cooled to room temperature, concentrated in vacuo, extracted with methylene chloride and NaOH aqueous solution, and the organic layer was dried over sodium sulfate and filtered. The residue was concentrated in vacuo to obtain dec-3-yn-1-yl 6-aminohexanoate with low purity. Without further purification, in a 500 mL 3-neck RBF, previously obtained dec-3-yn-1-yl 6-aminohexanoate, methylene chloride (130 mL), and triethylamine (4.33 g, 42.79 mmol, 2.20 eq) were added and cooled to 0 °C, and acryloyl chloride (1.94 g, 21.39 mmol, 1.10 eq) was added dropwise. The temperature of the reactor was raised to room temperature (20-25°C) and stirring was performed. After 18 hours, the mixture in the reactor was extracted with an HCl aqueous solution, and the organic layer was dried over sodium sulfate and filtered. The filtrate was concentrated in vacuo and purified using a silica column with EtOAc:hexane (1:2) to obtain dec-3-yn-1-yl 6-acrylamidohexanoate (765.60 mg, yield: 12%).
¹H-NMR (400 MHz, CDCl₃) δ 6.29 (d, 1H), 6.11 (m, 1H), 5.64 (d, 2H), 4.15 (t, 2H), 3.36 (t, 2H), 2.49 (m, 2H), 2.35 (t, 2H), 2.15 (t, 2H), 1.67 - 1.26 (m, 14H), 0.88 (t, 3H)

### 20-3. Synthesis of the compound of formula T

In a 100 mL 3-neck RBF, dec-3-yn-1-yl 6-acrylamidohexanoate (300.00 mg, 0.94 mmol, 2.20 eq), N,N'-dimethyl-1,3-propanediamine (43.80 mg, 0.43 mmol, 1.00 eq), and n-BuOH (5 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula T (165.3 mg, yield: 52%).
¹H-NMR (400 MHz, CDCl₃) δ 7.90 (s, 2H), 4.13 (t, 4H), 3.22 (q, 4H), 2.62 (t, 4H), 2.48 (m, 4H), 2.42 (m, 4H), 2.36 (t, 4H), 2.32 (t, 4H), 2.25 (s, 6H), 2.15 (m, 4H), 1.63-1.79 (m, 7H), 1.40-1.60 (m, 9H), 1.25-1.38 (m, 8H), 0.90 (t, 6H)

### Preparation Example 21

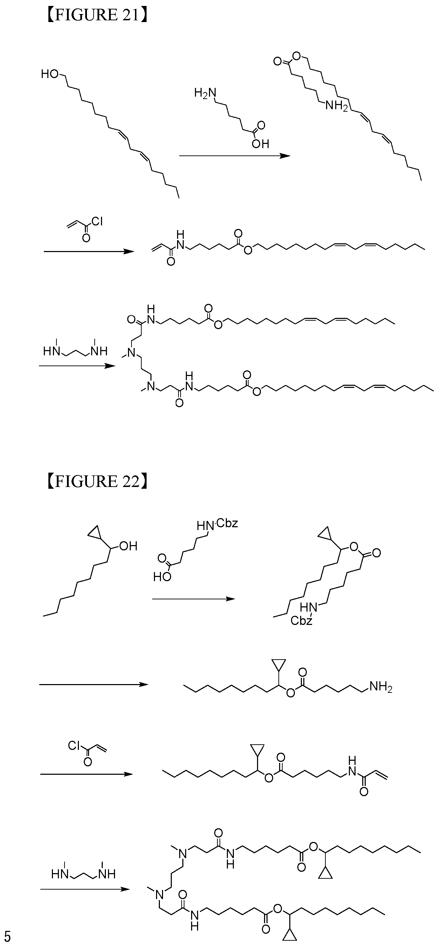

### 21-1. The compound of the following formula U was prepared according to the synthesis scheme shown in Figure 21.

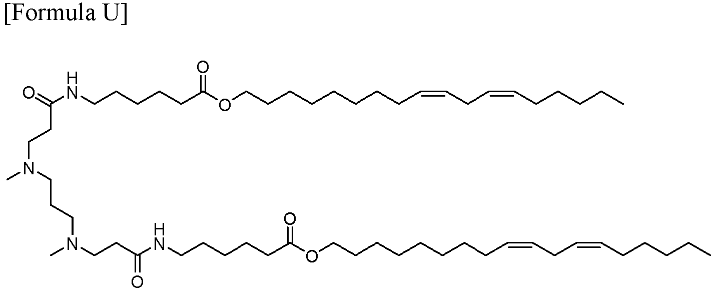

### 21-2. Synthesis of (9Z,12Z)-octadeca-9,12-dien-1-yl 6-acrylamidohexanoate

In a 100 mL 3-neck RBF, 6-aminohexanoic acid (295.37 mg, 2.25 mmol, 1.20 eq), (9Z,12Z)-octadeca-9,12-dien-1-ol (500.00 mg, 1.88 mmol, 1.00 eq), p-toluenesulfonic acid monohydrate (642.47 mg, 3.38 mmol, 1.80 eq), and cyclohexane (20 mL) were added, and a Dean-Stark trap and a condenser were installed and then stirring and reflux were performed. After 24 hours, the mixture was cooled to room temperature, concentrated in vacuo, extracted with methylene chloride and NaOH aqueous solution, and the organic layer was dried over sodium sulfate and filtered. The filtrate was concentrated in vacuo to obtain (9Z,12Z)-octadeca-9,12-dien-1-yl 6-aminohexanoate with low purity. Without further purification, previously obtained (9Z,12Z)-octadeca-9,12-dien-1-yl 6-aminohexanoate, methylene chloride (20 mL), and triethylamine (417.72 mg, 4.13 mmol, 2.20 eq) were added to a 100 mL 3-neck RBF and cooled to 0°C, and acryloyl chloride (186.82 mg, 2.06 mmol, 1.10 eq) was added dropwise. The temperature of the reactor was raised to room temperature (20-25°C) and stirring was performed. After 18 hours, the mixture in the reactor was extracted with an aqueous HCl solution, and the organic layer was dried over sodium sulfate and filtered. The filtrate was concentrated in vacuo and purified using a silica column with EtOAc:hexane (1:2) to obtain (9Z,12Z)-octadeca-9,12-dien-1-yl 6-acrylamidohexanoate (579.9 g, yield: 71%).
¹H NMR (400 MHz, CDCl₃) δ 6.27 (dd, 1H), 6.05-6.11 (m, 1H), 5.62-5.64 (m, 2H), 5.34-5.38 (m, 4H), 4.05 (t, 2H), 3.33 (q, 2H), 2.76 (t, 2H), 2.29 (t, 2H), 2.05 (q, 4H), 1.56-1.67 (m, 6H), 1.28-1.39 (m, 18H), 0.89 (t, 3H)

### 21-3. Synthesis of the compound of formula U

In a 100 mL 3-neck RBF, (9Z,12Z)-octadeca-9,12-dien-1-yl 6-acrylamidohexanoate (305.258 mg, 0.70 mmol, 2.40 eq), N,N'-dimethyl-1,3-propanediamine (30.00 mg, 0.29 mmol, 1.00 eq), and n-BuOH (3 mL) were added, and then stirring and reflux were performed. After 24 hours, the mixture was concentrated in vacuo at 80°C and purified using a silica column with methylene chloride:methanol:ammonium hydroxide (10:1:0.1) to obtain the compound of formula U (185.10 mg, yield: 65%).
¹H NMR (400 MHz, CDCl₃) δ 7.85 (s, 2H), 5.34-5.38 (m, 8H), 4.05 (s, 4H), 3.22 (q, 4H), 2.80 (t, 4H), 2.60 (t, 4H), 2.42 (t, 4H), 2.36 (t, 4H), 2.28 (t, 4H), 2.03 (s, 6H), 2.05 (q, 8H), 1.60-1.68 (m, 10H), 1.49-1.60 (m, 4H), 1.27-1.37 (m, 36H), 0.89 (t, 3H)

### Preparation Example 22

### 22-1. The compound of the following formula V was prepared according to the synthesis scheme shown in Figure 22.

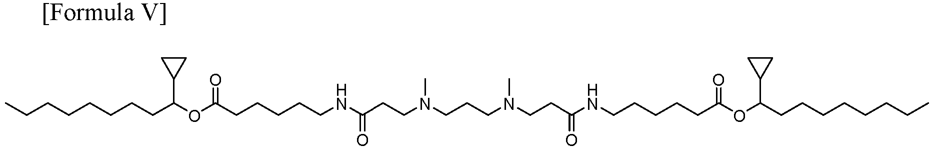

### 22-2. Synthesis of 1-cyclopropylnonyl 6-(((benzoyloxy)carbonyl)amino)hexanoate

In a 500 mL 3-neck RBF, 1-cyclopropylnonan-1-ol (8.68 g, 47.1 mmol, 1.00 eq) and methylene chloride (170 mL) were added at 25°C. 1-Ethyl-3-(3-dimethylaminopropyl)carbodiimide (EDCI) (11.7 g, 61.3 mmol, 1.30 eq) and triethylamine (Et3N) (9.54 g, 94.2 mmol, 2.00 eq) were added to the mixture. 6-(((benzyloxy)carbonyl)amino)hexanoic acid (15.0 g, 56.5 mmol, 1.20 eq) and 4-dimethylaminopyridine (DMAP) (1.15 g, 9.42 mmol, 0.20 eq) were added. The mixture was purged with nitrogen (N₂) three times. The mixture was stirred at 25°C for 16 hours. The reaction mixture was diluted with water (200 mL) and extracted with 600 mL of methylene chloride (200 mL x 3). The methylene chloride layer was collected, dried over sodium sulfate, filtered, and the filtrate was concentrated under reduced pressure to obtain the residue. The residue was purified by column chromatography (SiO₂, petroleum ether (PE)/ethyl acetate (EtOAc) = 10/1 to 1/100). 1-Cyclopropylnonyl 6-(((benzoyloxy)carbonyl)amino)hexanoate (9.00 g, 20.9 mmol, 44.3% yield) was obtained as a colorless oil.
¹H NMR (400 MHz, CDCl₃) δ 4.27 (td, 1H), 3.41 (t, 2H), 2.31 (t, 2H), 1.89 - 1.74 (m, 2H), 1.68 - 1.60 (m, 4H), 1.43 (s, 2H), 1.38 - 1.25 (m, 16H), 0.99 - 0.92 (m, 1H), 0.89 (t, 3H), 0.59 - 0.51 (m, 1H), 0.49 - 0.42 (m, 1H), 0.38 (qd, 1H), 0.31 - 0.21 (m, 1H)

### 22-3. Synthesis of 1-cyclopropylnonyl 6-aminohexanoate

Under argon (Ar) atmosphere, palladium catalyst (Pd/C) (2.22 g, 2.09 mmol, 10% purity, 0.10 eq) was added to a 35 mL portion of a cylindrical flask. Tetrahydrofuran (THF) (90 mL) was added to the top of the cylindrical flask. To the mixture, 1-cyclopropylnonyl 6-(((benzoyloxy)carbonyl)amino)hexanoate (9.00 g, 20.9 mmol, 1.00 eq) was added. Hydrogen (H₂) was charged to 50 psi. The mixture was stirred at 50°C for 16 hours. The Pd/C filter cake was safely filtered and collected. The filtrate was concentrated under pressure to obtain the residue. The residue was purified by column chromatography (SiO₂, methylene chloride/methanol = 10/1 to 1/100) to obtain 1-cyclopropylnonyl 6-aminohexanoate (6.00 g, 20.2 mmol, 96.7% yield) as a yellow oil.
¹H NMR (400 MHz, CDCl₃) *δ* 4.27 (td, 1H), 2.71 (t, 2H), 2.32 (t, 2H), 1.71 - 1.60 (m, 5H), 1.53 - 1.43 (m, 2H), 1.42 - 1.18 (m, 15H), 1.00 - 0.92 (m, 1H), 0.89 (t, 3H), 0.59 - 0.50 (m, 1H), 0.49 - 0.42 (m, 1H), 0.38 (qd, 1H), 0.33 - 0.18 (m, 1H)

### 22-4. Synthesis of 1-cyclopropylnonyl 6-acrylamidohexanoate

In a 100 mL 3-neck RBF, methylene chloride (30 mL) and 1-cyclopropylnonyl 6-aminohexanoate (3.00 g, 10.1 mmol, 1.00 eq) were added. To the mixture, triethylamine (TEA) (4.59 g, 45.4 mmol, 4.50 eq) was added at 0°C. To the mixture, acryloyl chloride (1.37 g, 15.1 mmol, 1.50 eq) was added dropwise at 0°C. The mixture was stirred at 0°C for 2 hours and then slowly returned to room temperature. The reaction mixture was neutralized by the addition of water (40 mL) at 20°C and extracted with methylene chloride (50 mL x 3). The combined organic layers were dried over sodium sulfate, filtered, and the filtrate was concentrated under reduced pressure to obtain the residue. The residue was purified by column chromatography (SiO₂, PE/EtOAc=10/1 to 1/100). 1-Cyclopropylnonyl 6-acrylamidohexanoate (2.60 g, 7.40 mmol, 73.3% yield) was obtained as a yellow oil.
¹H NMR (400 MHz, CDCl₃) *δ* 6.34 - 6.21 (m, 1H), 6.15 - 5.98 (m, 1H), 5.64 (br dd, 1H), 4.27 (td, 1H), 3.41 - 3.08 (m, 2H), 2.40 - 2.22 (m, 2H), 1.72 - 1.61 (m, 4H), 1.58 (s, 6H), 1.45 - 1.35 (m, 2H), 1.27 (br s, 9H), 1.00 - 0.91 (m, 1H), 0.89 (t, 3H), 0.61 - 0.51 (m, 1H), 0.50 - 0.41 (m, 1H), 0.37 (qd, 1H), 0.30 - 0.20 (m, 1H)

### 22-5. Synthesis of the compound of formula V

In a 50 mL 3-neck RBF, a mixture of dimethyl sulfoxide (DMSO) (3.5 mL) and water (3.5 mL), and 1-cyclopropylnonyl 6-acrylamidohexanoate (0.70 g, 1.99 mmol, 1.00 eq) were added. To the mixture, TEA (134 mg, 1.33 mmol, 0.67 eq) was added. To the mixture, N¹,N³-dimethylpropane-1,3-diamine (67.8 mg, 663 µmol, 0.33 eq) was added. The mixture was purged three times with N₂. The mixture was stirred at 100°C for 48 hours. The reaction mixture was concentrated under reduced pressure to obtain a residue. The residue was purified by column chromatography (SiO₂, methylene chloride/methanol=10/1) to obtain 0.3 g of a compound of formula V with relatively low purity. It was purified by reverse-phase HPLC (column: Phenomenex luna C18 15025 mm 10 µm; mobile phase: [water (FA)-ACN]; slope: 40%-70% B for 10 min) to obtain the compound of formula V (0.15 g, 186 µmol, 28.1% yield, 96.1% purity) as a yellow oil.
¹H NMR (400 MHz, CDCl₃) *δ* 7.91 (br s, 2H), 4.26 (td, 2H), 3.31 - 3.17 (m, 4H), 2.61 (t, 4H), 2.41 (t, 4H), 2.38 - 2.33 (m, 4H), 2.33 - 2.27 (m, 4H), 2.25 (s, 6H), 1.72 - 1.62 (m, 10H), 1.56 - 1.48 (m, 4H), 1.41 - 1.25 (m, 28H), 1.01 - 0.92 (m, 2H), 0.89 (t, 6H), 0.59 - 0.50 (m, 2H), 0.49 - 0.41 (m, 2H), 0.36 (qd, 2H), 0.31 - 0.21 (m, 2H)

### [Preparation of composition and test of delivery to lung]

### Example 1: Preparation of composition for drug delivery using the lipid of Preparation Example 2 and drug delivery test

### (1) Preparation of solutions for each component

The components shown in Table 1 below were dissolved in each dilution solvent to prepare their solutions at the concentrations shown in Table 1 below. When dissolving, a bath sonicator was used for about 5-10 minutes, and the solutions were used after visually confirming that there were no undissolved particles. For dioleoyl phosphatidylethanolamine (DOPE) and cholesterol, the solutions were incubated in an oven at 65°C for about 5 minutes and used in the test after visually confirming that there was no precipitation.

**[Table 1]**

| **No.** | **Components** | **Dilution solvents** | **Concentration for use** |
|---|---|---|---|
| 1 | **mRNA** | RNAse free water | 1 mg/mL |
| 2 | **Lipid of Preparation Example 2** | Ethanol 100% | 10 - 20 mg/mL |
| 3 | **mPEG-PLA(2K-4K)** | Ethanol 95% | 50 - 100 mg/mL |
| 4 | **DOPE** | Ethanol 100% | 10 - 20 mg/mL |
| 5 | **Cholesterol** | Ethanol 100% | 10 - 20 mg/mL |

| | | | |
|---|---|---|---|
| [mPEG-PLA(2K-4K): Copolymer of monomethoxypolyethylene glycol (mPEG) block with a number average molecular weight of 2,000 and polylactic acid (PLA) block with a number average molecular weight of 4,000] | | | |

### (2) Preparation of composition

The required amounts of the components were mixed in order to meet the N/P ratio (amine group of lipid component/phosphate group of mRNA) of 20 and Lipid of Preparation Example 2:mPEG-PLA(2K-4K):DOPE:cholesterol=5:5:1:4. Ethanol was added to the ethanol layer so that the molecular total of all components was 6.25-12.5 mM, and the aqueous phase and ethanol phase were mixed maintaining a ratio of 3:1. After mixing, the total amount was diluted 20 times with 1XPBS to lower the total ethanol content, and then concentrated using Amicon-Ultra tube filter (Merk Millipore, UFC505096 or UFC805024, pore size: 50K or 100K, volume: 0.5 mL or 4 mL). The buffer used in the aqueous phase was 20 mM sodium acetate buffer (pH 4.6) (This was prepared by diluting 3M sodium acetate buffer to 20 mM and titrating it to pH 4.6 using 1M HCl). The more concrete procedure of composition preparation is as follows:
1) Two autoclaved tubes were prepared (tubes (A) and (B)).
2) In tube (A), Lipid of Preparation Example 2, DOPE, cholesterol, and mPEG-PLA (2K-4K) in molar quantities calculated according to the experimental conditions were sequentially added and mixed by vortexing.
3) In the ethanol phase, when necessary, ethanol was added so that the molecular total of all components was within 6.25-12.5 mM.
4) In the case of mPEG-PLA, since at least 3% of water was required for complete dissolution, water was added for dissolution so that the total ethanol phase became 95% ethanol.
5) In tube (B), mRNA and 20 mM sodium acetate buffer (pH 4.6) were mixed. At that time, the ratio was calculated and added so that the aqueous phase was total three times the amount of the ethanol phase.
6) The solution in tube (B) was transferred to tube (A) and mixed as illustrated below. At that time, the mixing was performed as quickly as possible to make a uniform formulation, and then vortexing was performed for mixing. 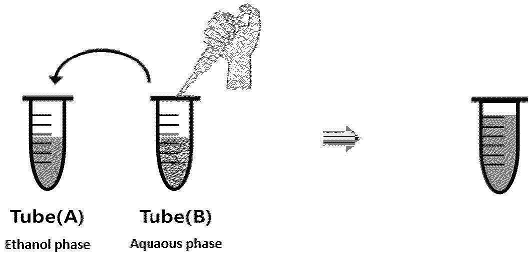
7) To the prepared formulation, PBS was added for dilution to make the ethanol content 5% or less.

### (3) Concentration and sterilization

1) The diluted formulation was centrifuged using Amicon Ultra Centrifugal Filter until it was concentrated to the desired volume depending on the purpose.
2) Once the formulation was concentrated to the desired concentration, it was sterilized using a 0.22 µm pore size filter.

### (4) Administration of composition

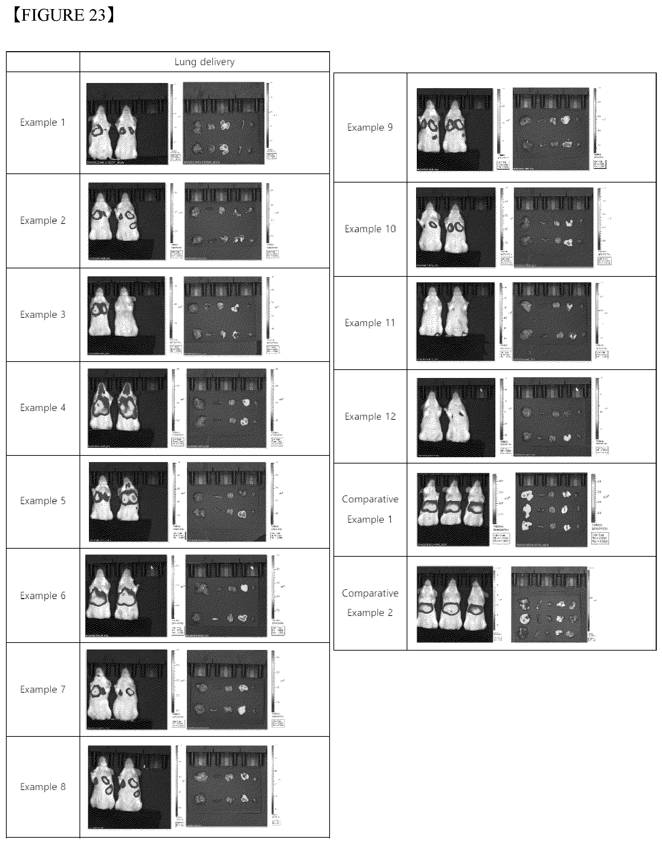

The formulation prepared according to the above method was prepared at 10 µg/mL, and administered intravenously to mice so that 2 µg based on mRNA was administered per mouse. After 4 hours, luciferin dissolved in sterile water was prepared at 15 µg/µL and administered intraperitoneally so that 3 mg of luciferin was administered per 20 g mouse. After 15 minutes from the intraperitoneal administration of luciferin, the results of protein expression for whole body and each organ were measured using a luminescence measurement imaging system and are shown in Table 2 and Figure 23.

As confirmed in Figure 23, the drug delivery formulation according to the present invention had very excellent delivery efficiency to lung when administered intravenously.

Meanwhile, as comparative examples, LNPs were prepared by using SM102 [Heptadecan-9-yl 8-((2-hydroxyethyl)(6-oxo-6-(undecyloxy)hexyl)amino)octanoate, purchased from SINOPEG] and D-Lin-MC3-DMA [dilinoleylmethyl-4-dimethylaminobutyrate, purchased from MedChemExpress], and administered intravenously to mice with the same amount of mRNA (2µg/200µL) as in the example. After 4 hours, imaging was performed by the same method as in the example, and the results are shown in Table 2 and Figure 23. As confirmed in Figure 23, the formulations of the comparative examples were delivered to liver when administered intravenously.

### Examples 2 to 12: Preparation of composition for drug delivery using each lipid of Preparation Examples 4, 5, 7, 8, 9, 10, 12, 13, 14, 15 and 16, respectively, and drug delivery test to lung

Compositions for drug delivery were prepared by using each of the lipids of Preparation Examples 4, 5, 7, 8, 9, 10, 12, 13, 14, 15 and 16, respectively, instead of the lipid of Preparation Example 2, in the same manner as in Example 1 at an N/P ratio shown in Table 2 below. By using the compositions, drug delivery tests were performed in the same manner as in Example 1, and the results are shown in Table 2 and Figure 23.

As confirmed in Figure 23, the drug delivery formulations of Examples 2 to 12 according had very excellent delivery efficiency to lung when administered intravenously as in Example 1.

**[Table 2]**

| | Lipid | N/P ratio | Lung Avg Radiance [p/s/cm²/sr] |
|---|---|---|---|
| Example 1 | Lipid of Preparation Example 2 | 20 | 1.12E+05 |
| Example 2 | Lipid of Preparation Example 4 | 20 | 9.13E+04 |
| Example 3 | Lipid of Preparation Example 5 | 20 | 1.15E+06 |
| Example 4 | Lipid of Preparation Example 7 | 20 | 1.35E+06 |
| Example 5 | Lipid of Preparation Example 8 | 20 | 2.49E+07 |
| Example 6 | Lipid of Preparation Example 9 | 20 | 4.01E+05 |
| Example 7 | Lipid of Preparation Example 10 | 20 | 1.51E+06 |
| Example 8 | Lipid of Preparation Example 12 | 20 | 7.06E+05 |
| Example 9 | Lipid of Preparation Example 13 | 20 | 3.99E+06 |
| Example 10 | Lipid of Preparation Example 14 | 20 | 2.42E+05 |
| Example 11 | Lipid of Preparation Example 15 | 20 | 3.59E+04 |
| Example 12 | Lipid of Preparation Example 16 | 20 | 4.27E+04 |
| Comparative Example 1 | SM102 | 5.2 | 2.02E+8 |
| Comparative Example 2 | D-Lin-MC3-DMA | 3 | 3.08E+7 |

## Claims

1. A composition for drug delivery comprising:
drug as effective ingredient; and
a lipid having a structure selected from the following, or an ionized form thereof: 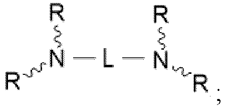 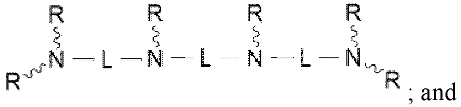 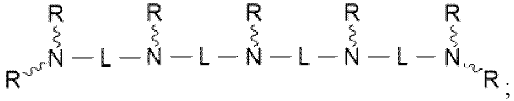
wherein, in each of the above structures,
at least two of R groups are Rx, and other R groups are Ry, wherein
each Rx is independently selected from 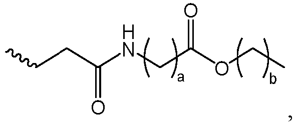 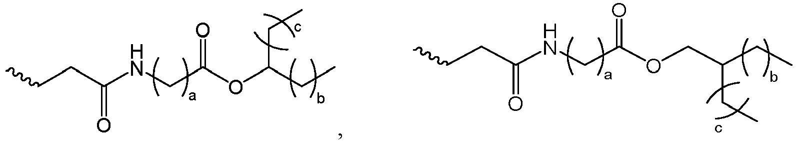 and 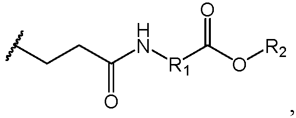 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 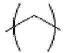 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

2. The composition for drug delivery according to claim 1, wherein
each Rx is independently selected from 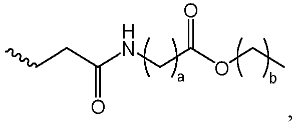 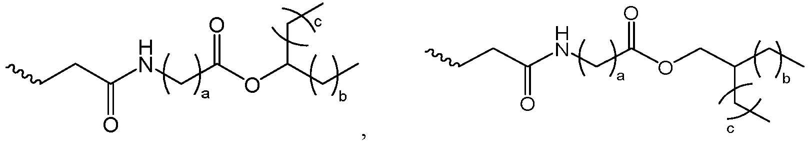 and 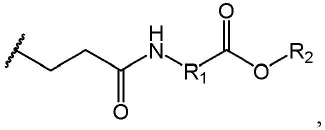 where each of a, b and c is independently an integer of from 2 to 20, R₁ is substituted or unsubstituted, saturated or unsaturated divalent C₁₋₁₂ hydrocarbon group, R₂ is substituted or unsubstituted, unsaturated monovalent C₂₋₂₄ hydrocarbon group, and 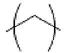 represents substituted or unsubstituted methylene group;
each Ry is independently H or C₁₋₂₀ alkyl group, where the alkyl group is independently unsubstituted, or substituted with one or more selected from -OH, C₁₋₂₀ alkyl, C₁₋₂₀ alkoxy, -NH₂, -NH(C₁₋₂₀ alkyl), -N(C₁₋₂₀ alkyl)₂, optionally substituted C₃₋₂₀ carbocyclic group and optionally substituted C₃₋₂₀ heterocyclic group, where two Ry groups that are not H may be connected together with nitrogen atom to which they are attached, to form a ring structure; and
each L is independently C₁₋₂₀ alkylene group, each of which is independently unsubstituted, or substituted with one or more selected from -OH, C₁₋₂₀ alkyl, C₁₋₂₀ alkoxy, -NH₂, - NH(C₁₋₂₀ alkyl), -N(C₁₋₂₀ alkyl)₂, optionally substituted C₃₋₂₀ carbocyclic group and optionally substituted C₃₋₂₀ heterocyclic group.

3. The composition for drug delivery according to claim 2, wherein
each Rx is independently selected from 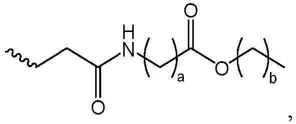 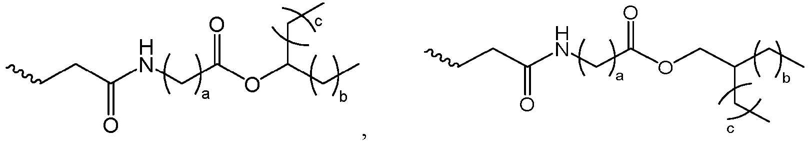 and 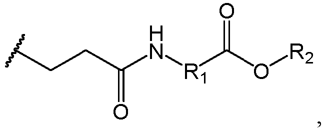 where each of a, b and c is independently an integer of from 2 to 15, R₁ is substituted or unsubstituted, saturated or unsaturated divalent C₁₋₁₂ hydrocarbon group, R₂ is substituted or unsubstituted, unsaturated monovalent C₂₋₂₄ hydrocarbon group, and 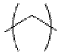 represents substituted or unsubstituted methylene group;
each Ry is independently H or C₁₋₁₀ alkyl group, where the alkyl group is independently unsubstituted, or substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group, where two Ry groups that are not H may be connected together with nitrogen atom to which they are attached, to form a ring structure; and
each L is independently C₁₋₁₀ alkylene group, each of which is independently unsubstituted, or substituted with one or more selected from -OH, C₁₋₁₀ alkyl, C₁₋₁₀ alkoxy, -NH₂, -NH(C₁₋₁₀ alkyl), -N(C₁₋₁₀ alkyl)₂, optionally substituted C₃₋₁₀ carbocyclic group and optionally substituted C₃₋₁₀ heterocyclic group.

4. The composition for drug delivery according to claim 3, wherein
each Rx is independently selected from 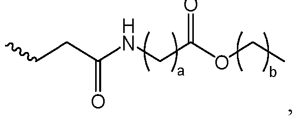 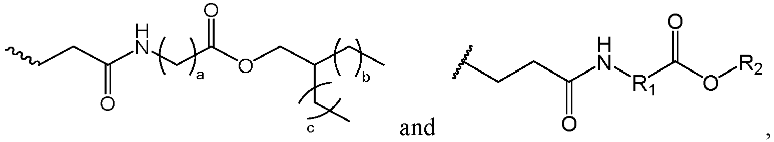 where each of a, b and c is independently an integer of from 3 to 12, R₁ is substituted or unsubstituted C₁₋₁₂ alkylene group, substituted or unsubstituted C₂₋₁₂ alkenylene group or substituted or unsubstituted C₂₋₁₂ alkynylene group, R₂ is substituted or unsubstituted C₂₋₂₄ alkenyl group or substituted or unsubstituted C₂₋₂₄ alkynyl group, and 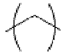 represents substituted or unsubstituted methylene group;
each Ry is independently H or C₁₋₆ alkyl group, where the alkyl group is independently unsubstituted, or substituted with one or more selected from -OH and -NH₂; and two Ry groups that are not H may be connected together with nitrogen atom to which they are attached, to form a ring structure; and
each L is independently unsubstituted C₁₋₆ alkylene group.

5. The composition for drug delivery according to claim 4, wherein the lipid is one having a structure selected from the following formulas A to V:
| Formula | Structure |
|---|---|
| A | 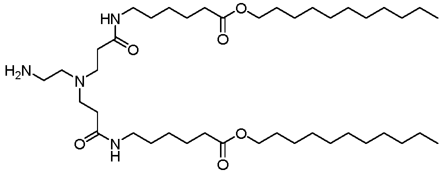 |
| B | 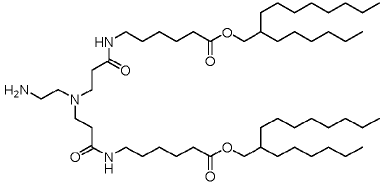 |
| C | 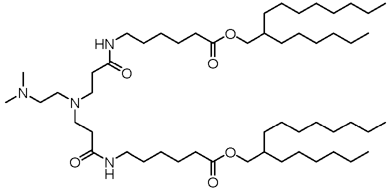 |
| D | 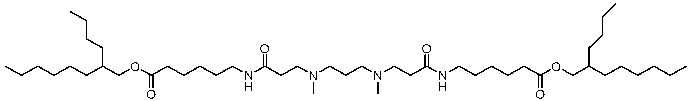 |
| E | 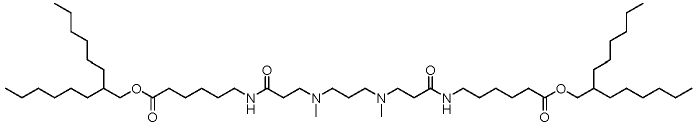 |
| F | 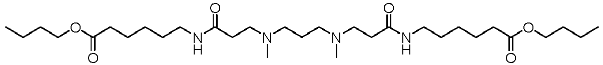 |
| G | 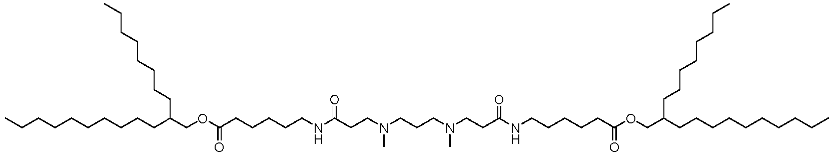 |
| H | 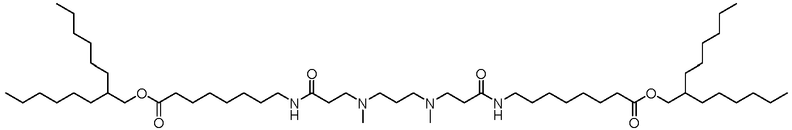 |
| I | 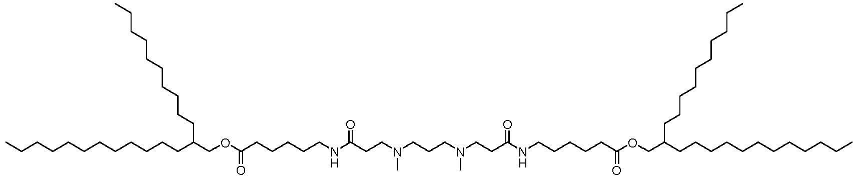 |
| J | 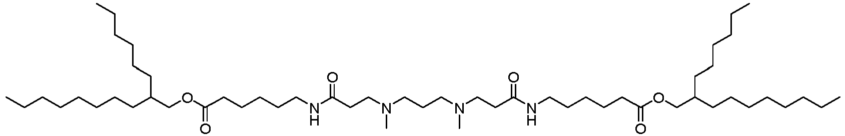 |
| K | 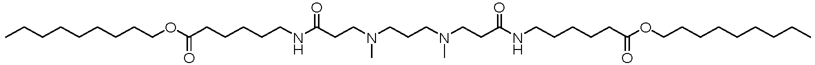 |
| L | 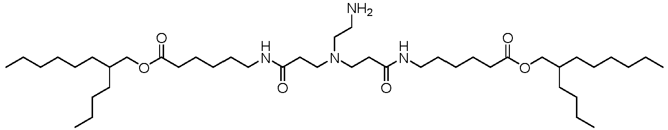 |
| M | 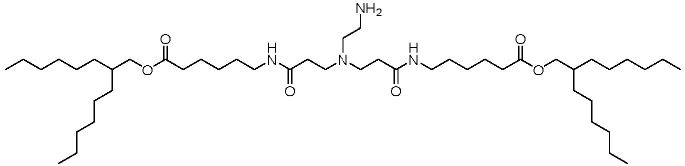 |
| N | 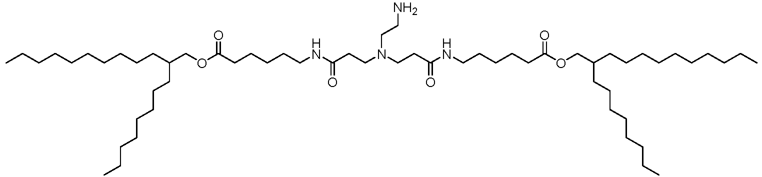 |
| O | 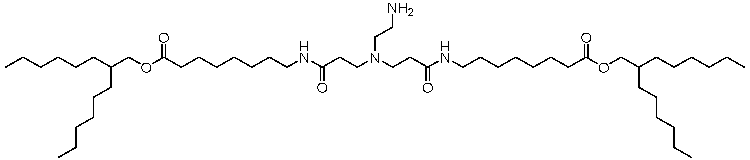 |
| P | 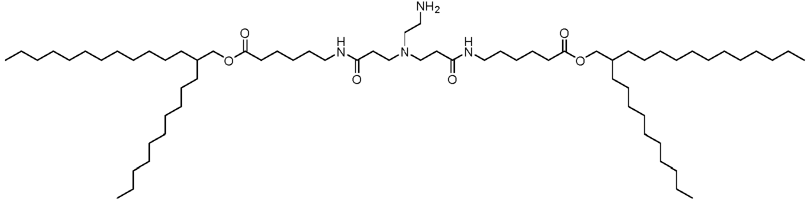 |
| Q | 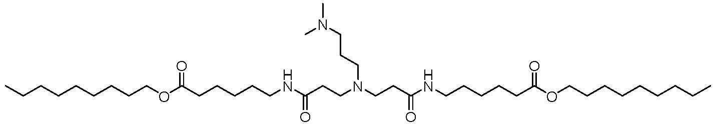 |
| R | 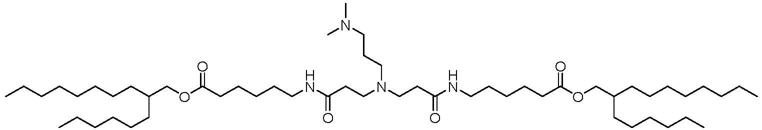 |
| S | 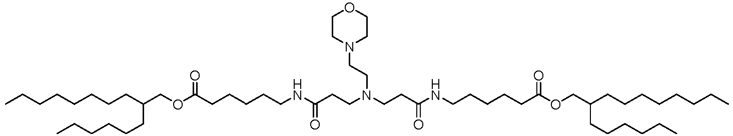 |
| T | 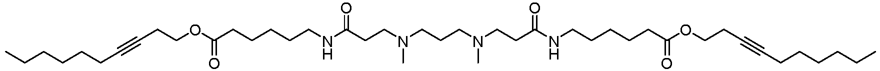 |
| U | 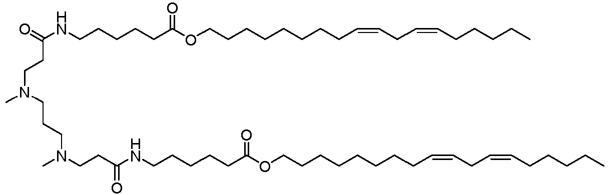 |
| V | 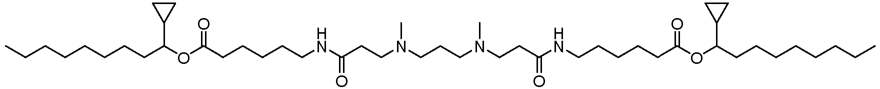 |

6. The composition for drug delivery according to claim 1, wherein the drug forms a complex with the lipid.

7. The composition for drug delivery according to claim 1, wherein the composition further comprises amphiphilic block copolymer.

8. The composition for drug delivery according to claim 7, wherein the drug forms a complex with the lipid, and the complex is encapsulated within nanoparticle structure formed by the amphiphilic block copolymer.

9. The composition for drug delivery according to claim 1, wherein the composition is in a form of kit which comprises a first chamber comprising the lipid; and a second chamber comprising the drug.

10. The composition for drug delivery according to claim 9, wherein the first chamber further comprises amphiphilic block copolymer.

11. A composition for drug delivery comprising nanoparticle,
wherein the nanoparticle does not comprise drug, and
wherein the nanoparticle comprises a lipid having a structure selected from the following,
or an ionized form thereof: 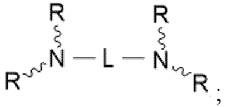 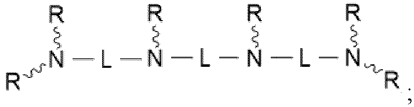 and 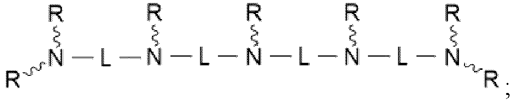
wherein, in each of the above structures,
at least two of R groups are Rx, and other R groups are Ry, wherein
each Rx is independently selected from 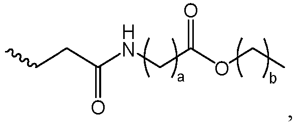 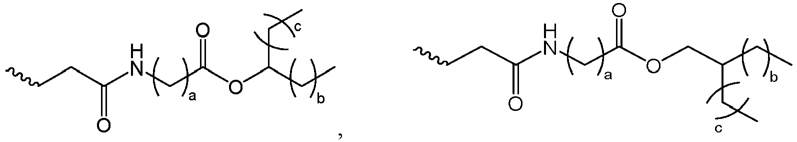 and 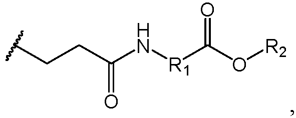 where each of a, b and c is independently an integer of from 2 to 20, R₁ is substituted or unsubstituted, saturated or unsaturated divalent hydrocarbon group, R₂ is substituted or unsubstituted, unsaturated monovalent hydrocarbon group, and 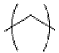 represents substituted or unsubstituted methylene group, and
each Ry is independently H, or substituted or unsubstituted alkyl group, where two Ry groups that are not H may be connected together with nitrogen atom to which they are attached, to form a ring structure; and
each L is independently substituted or unsubstituted alkylene group, and may have in its structure optionally ether bond (-O-), thioether bond (-S-) or disulfide bond (-S-S-).

12. The composition for drug delivery according to claim 11, wherein the nanoparticle further comprises amphiphilic block copolymer.

13. The composition for drug delivery according to any one of claims 1 to 12, wherein the composition is for delivering drug to lung.

14. The composition for drug delivery according to any one of claims 1 to 12, wherein the composition further comprises salt of polylactic acid.
